# EUROPEAN PATENT APPLICATION

(11) **EP 0 758 681 A2**
(43) Date of publication of application: **19.02.1997**
(21) Application number: 96201406.4
(22) Date of filing: 14.02.1990
(51) Int. Cl.: C12N 15/00

(54) **Inhibiting transformation of cells having elevated purine metabolic enzyme activity**

(30) Priority: 14.02.1989 US 310773; 28.04.1989 US 344963; 18.01.1990 US 467147
(62) Divisional of application: 90904105.5
(71) Applicant: MASSACHUSETTS INSTITUTE OF TECHNOLOGY, Cambridge, MA 02139 (US); THE GENERAL HOSPITAL CORPORATION, Charlestown, MA 02129 (US); HARVARD UNIVERSITY, Cambridge Massachusetts 02138-5701 (US); AMIRA INC., Cambridge, Massachusetts 02142 (US)
(72) Inventor: Kaddurah-Daouk, Rima, Watertown, MA 02172 (US); Daouk, Ghaleb, Watertown, MA 02172 (US); Kingston, Robert, Brighton, MA 02135 (US); Green, Michael, somerville, MA 02143 (US); Schimmel, Paul, Lexington, MA 02173 (US); Lillie, James W., Somerville, MA 02143 (US); Putney, Scott D., Arlington, MA 02174 (US)
(74) Representative: Holdcroft, James Gerald, Dr.

(57) **Abstract**

A method of inhibiting growth rate, transformation or metastasis of mammalian cells, particularly epithelial cells, in which activity of at least one enzyme which participates in purine metabolism and regulations of nucleotide levels is elevated. In particular, a method of inhibiting transformation of mammalian cells by a DNA tumor virus, a DNA tumor virus factor or other factor which has an equivalent effect on cells.

## Description

### Background

Transformation, or malignant transformation, of cells results in changes in their growth characteristics and can cause them to form tumors in animals into whom they are introduced. For example, transformation of adherent cells can be associated with alterations such as changes in growth control, cell morphology, membrane characteristics, protein secretion and gene expression. Although transformation can occur spontaneously, it can be caused by a chemical or irradiation or may result from infection by a tumor virus. Little is known about the underlying molecular events. One type of RNA viruses (retroviruses) and many different types of DNA viruses can act to transform cells and collectively are referred to as tumor viruses. In the case of tumor viruses, it is clear that the virus does not itself carry all of the genes necessary to produce the phenotypic changes characteristic of infected cells. Tumor viruses may act through a gene or genes in their genome (oncogenes) which, in some way, influence or induce target cell genes. The induced target cell genes, in turn, act to carry out the changes observed in transformed cells. There are at least three major classes of transforming DNA viruses: adenoviruses, which have two groups of oncogenes, E1A and E1B, which act together to produce transformation; papovaviruses, which synthesize proteins, called T antigens, which may work together to transform cells; and herpes viruses, for which no oncogene has been identified as yet.

Although considerable effort has been expended in identifying transforming genes or oncogenes and, in some cases, has also resulted in identification of their protein products, very little is known about the cellular mechanisms affected in the transformation process. There is a consensus that these oncogenes perturb cell growth by modifying the expression or activity of key growth related genes. It would be very helpful to have a better understanding of how transformation occurs, particularly if the biochemical pathways affected can be identified. Such knowledge would make it possible to design compounds which can interfere with or counter the effects of the transforming or minimizing the exent to which it occurs once begun and thus to reduce effects on individuals in whom it occurs.

### Disclosure of the Invention

The present invention relates to a method of inhibiting growth rate, transformation or metastasis of mammalian cells, particularly epithelial cells, in which activity of at least one enzyme which participates in purine metabolism and regulation of nucleotide levels (a purine metabolic enzyme) is elevated. In particular, it relates to a method of inhibiting (reducing or eliminating) transformation of mammalian cells by a DNA tumor virus, a DNA tumor virus factor or other factor which has an equivalent effect on cells (i.e., causes, either directly or indirectly, an increase in purine metabolic enzyme activity). The method is carried out by inhibiting or interfering with the increase in host gene expression caused by the DNA tumor virus or factor, particularly by interfering with the ability of the virus or the factor to increase expression of a gene(s) which encodes host cell purine metabolic enzyme(s). It relates to a method or reducing cellular transformation by interfering with the ability of an oncogenic product(s) of a DNA tumor virus or of other cellular activated proteins with functions similar to those of oncogenic products of a virus associated with transformation, to increase expression of host cell purine metabolic enzyme gene expression. If further relates to compounds or drugs useful in reducing the effects on cells of a DNA tumor virus oncogenic product. The present invention further relates to a method of inhibiting replication of a DNA tumor virus in cells in which purine metabolic enzyme activity is increased and to a method of inhibiting viral activity.

Drugs useful in the present method can have the desired effect on cell transformation by acting in one or more of the following ways: by inhibiting the purine metabolic enzyme whose activity in the cell is elevated; by decreasing the message level; by interfering with the transcription factors which induce expression of the host cell purine metabolic enzymes; and by inhibiting interaction of the viral product or the factor with a host cell gene(s)s encoding a purine metabolic enzyme(s). Alternatively, the drugs may act by inhibiting interaction of the viral product or factor with a host cell gene product(s), the interaction of which regulates the activity of the purine metabolic enzymes, or their expression. As antiviral agents, such drugs act by interfering with the replication of the virus.

Drugs useful in the present method can be existing drugs, analogues of existing drugs or drugs designed specifically for the purpose of reducing purine metabolic enzyme activity, such as by inhibiting the DNA tumor virus or its activity.

Through use of the present method, it is possible to reduce or eliminate the transforming ability of DNA tumor viruses and, thus, to reduce the extent to which tumor formation occurs or to prevent tumor formation.

Other tumors which are characterized by high levels of expression of the purine metabolic enzymes and which are associated directly or indirectly with the presence of the DNA tumor virus can be treated in a similar fashion. In addition, tumors that result from cellular mutations that mimic the effects of infection by a DNA tumor virus can be treated in a similar manner. For instance, loss of antioncogene products Rb, DCC or p53 may mimic infection by a DNA tumor virus, leading to high levels of expression of purine metabolic enzymes. These tumors are likely candidates for treatment by the present methods.

A genomic clone for the human creatine kinase B-isozyme has been isolated and the gene has been shown to encode a functional enzyme, as demonstrated by transfection of the cloned DNA into different cell lines and subsequent observation of the production of the B-isozyme. The promoter has been shown to have a strong sequence relationship with the promoter region of the adenovirus E2E gene. The E2E region of the adenovirus encodes a 72 kd single stranded DNA binding protein which is involved in the replication of the virus. Expression of the E2 gene and subsequent viral replication, is highly dependent on the oncogenic products of the adenovirus E1a region.

The CKB promoter region provides the first example of a strong sequence similarity between a cellular gene promoter region and a viral gene promoter. The strong similarity to the first 100 nucleotides of the 5'-noncoding sequence of the E2 promoter suggests that expression from each promoter is regulated by some factors common to both. This model is supported by the srikingly good alignment of the two promoters through each of the four sub-regions of the promoter that are important for expression of E2. (Some or all of the first 100 nucleotides of the adenovirus E2E promoter are required for its expression). This surprising relationship of the two promoter sequences motivated an investigation for a possible functional relationship between the two. As described herein, it has been shown that brain creatine kinase expression is regulated by the oncogenic products of the E1a region of the adenovirus. Thus, for the first time, it has been shown that a cellular gene associated with energy metabolism and regulation of intracellular nucleotide levels is turned on, or activated, by the transforming domains of an oncogene. That is, induction of a cellular gene by oncogenic products of a transforming virus has been demonstrated. In particular, regulation of brain creatine kinase expression by the short (19 amino acid) transforming domain 2 of the E1a oncogene has been shown. This is of interest because a whole range of human transforming viruses (e.g. adenoviruses, SV40, papilloma virus) has been shown to contain transforming domains close in sequence to domain 2. This same domain in the protein has also recently been shown to interact with the cellular anti-oncogene product of the retinoblastoma gene, revealing a novel oncogene-anti-oncogene association. Domain one of E1a, which is also involved in transformation and induction of DNA synthesis, is also important for the induction of CKB. Cyclin, which is a cell cycle regulated gene, important for cell division, has also been shown to require this domain. It is reasonable to suggest that CKB is part of the trigger mechanism in cell division and for growth of some malignant cells.

The induction of a cellular gene that is important for regulating energy metabolism and nucleotide levels of the cell by the transforming domains might be important for regulation of intermediary metabolic events that take place after oncogenic activation. Blocking the activity of creatine kinase, which is a tumor marker for small cell lung carcinoma (SCLC), active in many malignancies and which is shown to be induced by the DNA tumor virus or its cellular homologue in transformed cells, will be very useful for controlling the growth of the tumor or extent of transformation, as well as the ability of the viruses to replicate. Of great significance will be the ability to interfere with human DNA viruses, such as the human papilloma virus, which is associated with malignancies of the cervix. These viruses encode oncogenic molecules, such as the E7 product of HPV-16, which is similar structurally and functionally to the E1a product of adenovirus in the transformation domains. The human cytomegatovirus which is known to induce CKB levels, the oncogenic molecules or the cellular analogues, regulates similar metabolic events important for transformation of the affected cell. Creatine kinase, along with some enzymes that work in conjunction with it, are part of the important events leading to transformation. Inhibiting the activity of CK and associated enzymes will be very useful in controlling transformation and the replication of the viruses.

For the first time, the promoters of two cellular genes--the brain creatine kinase gene and the adenosine deaminase gene--each of which participates in cellular nucleotide metabolism and is naturally overexpressed in tumors--have been shown to have remarkable sequence similarity.

A link between the functions of these enzymes involved in purine metabolism and nucleotide level regulation and the replication gene in a DNA tumor virus plus their indiction by the transforming molecules of the DNA tumor virus suggests an important function they mediate in DNA tumor viral functions.

Regulating adenosine pools by creatine kinase and associated enzymes might affect guanine pools directly or indirectly. It has been demonstrated that there is a link beteen the conversion of guanidine diphosphate (GDP) to guanidine triphosphate (GTP) through ATP regulation through creatine kinase. Bessman, S.P. & L.C. Carpenter, Ann. Rev. Biochem., 54:831.862 (1985). The regulation of GDP/GTP pools is vital for many processes activated during transformation. The ras oncogene product (p21) associated with many malignancies is regulated by GDP/GTP pools, as well as a family of signally G-proteins and micro tubules assembly. Hence the creatine kinase system which is induced by a nuclear oncogene might link nuclear events to cycloplasmic events activated during malignancy.

Hence again regulating the activity of the creatine kinase system and the enzymes working in conjunction with it will control cytoplasmic events necessary to sustain transformation or viral entry and replication. The link now shown to exist between the cellular enzymes involved in early uptake and metabolism of adenosine to effects of DNA tumor viruses and transformation provides an excellent basis for drug design. Compounds active in regulating these enzymes and others working with them will be of utmost importance in chemotherapy strategies and in antiviral drug strategies. Drugs used in the method of the present invention will act by interfering with the effect of the viral product on expression of the gene encoding the key cellular enzyme; interfering with the activity of the activated or induced enzyme; interfering with the activity of other components of adenosine uptake and its metabolic pathways or the signals for their activation; or by interfering with any combination of the three. For example, expression of transforming domain 2 of the E1a region of an adenovirus, now known to induce creatine kinase expression in cells, can be inhibited. Similarly the region around domain one of E1a also needed for induction of CKB and cyclin can be interfered with. For example, creatine kinase catalyzes the reversible storage of ATP as creatine phosphate, and its regeneration to maintain a high ATP/ADP ratio in the cell. The level of brain creatine kinase is greatly enhanced in tumor cells, such as in small cell lung carcinoma. The method of the present invention can be used to regulate (e.g., inhibit or interfere with) the activity of brain creatine kinase, and, thus, is useful in controlling the growth of tumor cells. For example, other components of the adenosine metabolic pathway, or signals for its activation, can be inhibited or interfered with, thereby modifying nucleotide levels and distribution and DNA synthesis. Thus, the invention provides a method for countering oncogenesis and viral-induced cell proliferation by controlling the levels of ATP available for cell metabolism, growth, and generation of secondary messengers.

### Brief Description of the Drawings

Figure 1 is a partial restriction map of the human genomic clone 16B2. The cross-hatched area represents the 2.5kbp coding region within the human creatine kinase B isozyme gene.

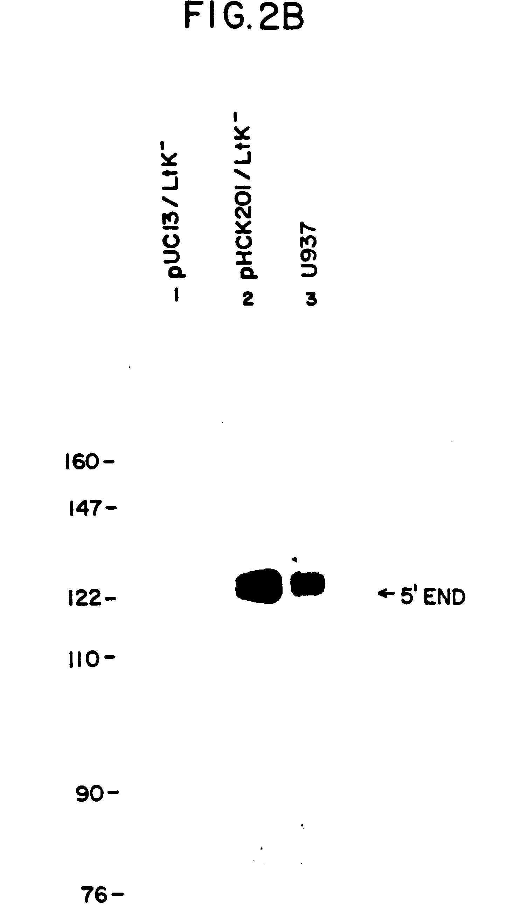

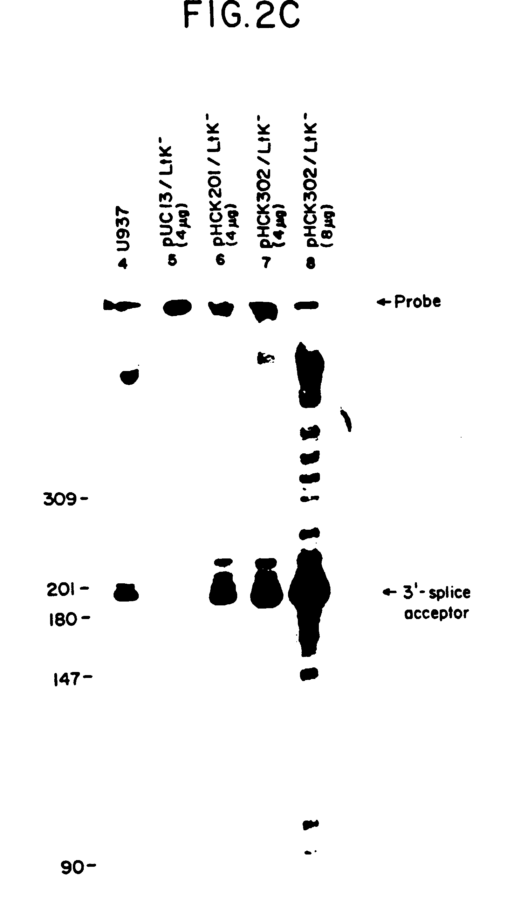

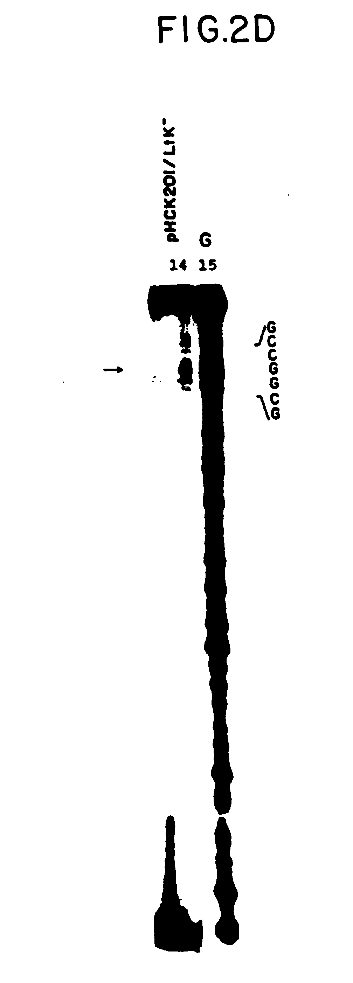

Figure 2 shows mapping of the transcriptional start site of the human gene for brain creatine kinase. Panel A is a simple map at the 5' end of the gene for the B isozyme and the locations of probes and oligonucleotides for analysis described herein. The arrow indicates the position of the 5' end of the mRNA that was determined by this analysis. Panel B shows results of primer extension mapping of the 5' end of the mRNA for the human B isozyme gene. Panel C shows results of S1 nuclease mapping of the 5' end of the mRNA. Panel D shows results of S1 nuclease mapping with synthetic oligomer 3 (a 60-mer synthetic oligomer).

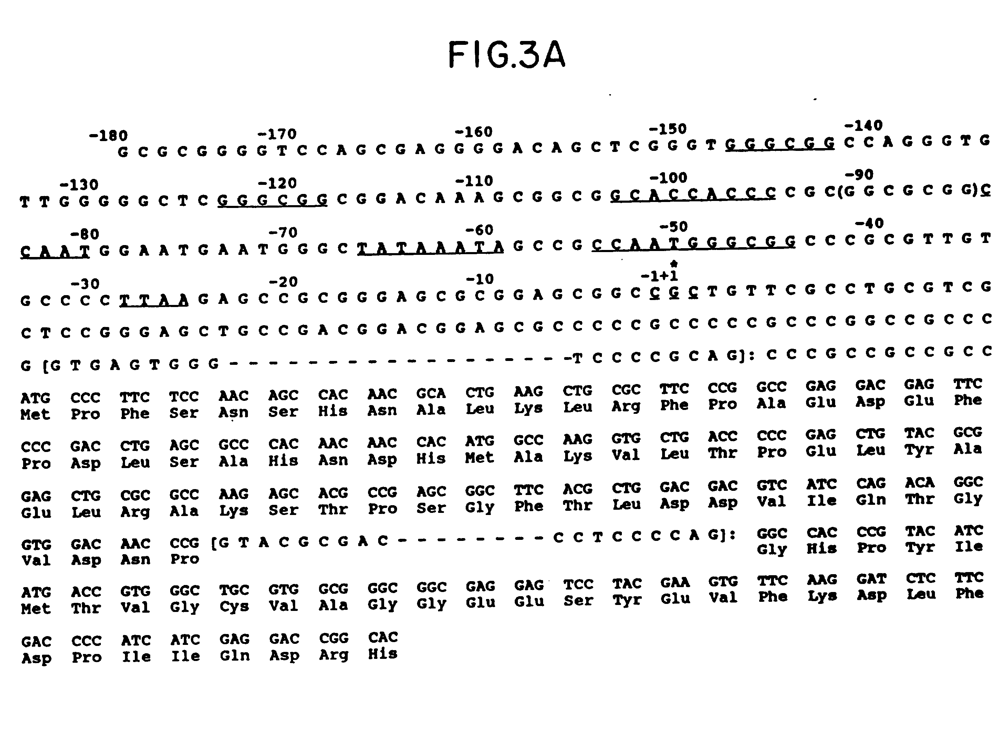

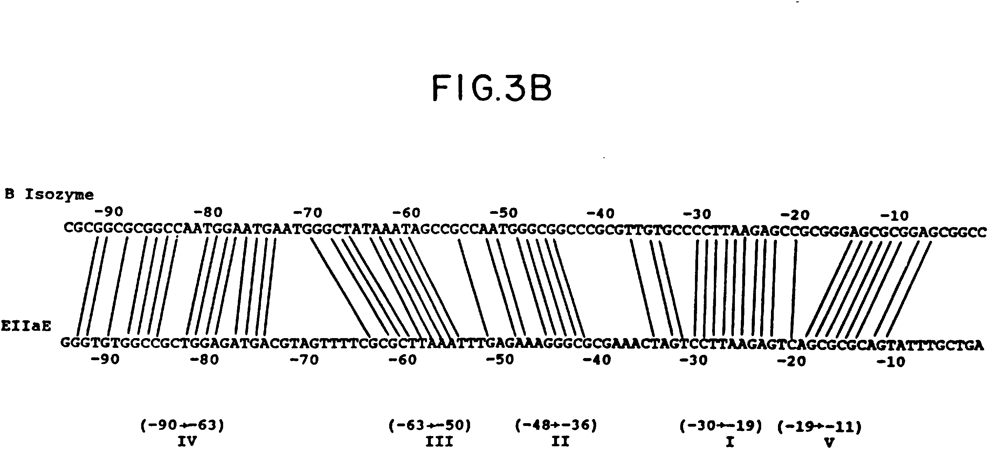

Figure 3 is the nucleotide sequence of the human B isozyme of creatine kinase. Panel A is the sequence of the amino-terminal coding region and of the 5'-flanking sequences. Parentheses enclose the -90 to -84 heptanucleotide sequence which behaved aberrantly in the sequencing of both strands and may contain an error. The asterisk indicates the location in the gene of the major 5'-end of the transcript that was determined by primer extension analysis and S1 nuclease mapping experiments. Nucleotides are numbered consecutively from this site. Brackets indicate the positions of the first two introns. Underlines have been used to highlight sequence elements that are typically associated with promoters or with binding sites for regulatory proteins. Panel B, sequence relationship between the adenovirus E2 promoter and the 5'-region of the B isozyme of creatine kinase. Lines connect nucleotides that are identical. Regions I-IV of the adenovirus E₂E sequence have been designated as important for expression (20,40), and similar regions are found in the gene for the B isozyme. A fifth region (designated V) of sequence similarity is also noted.

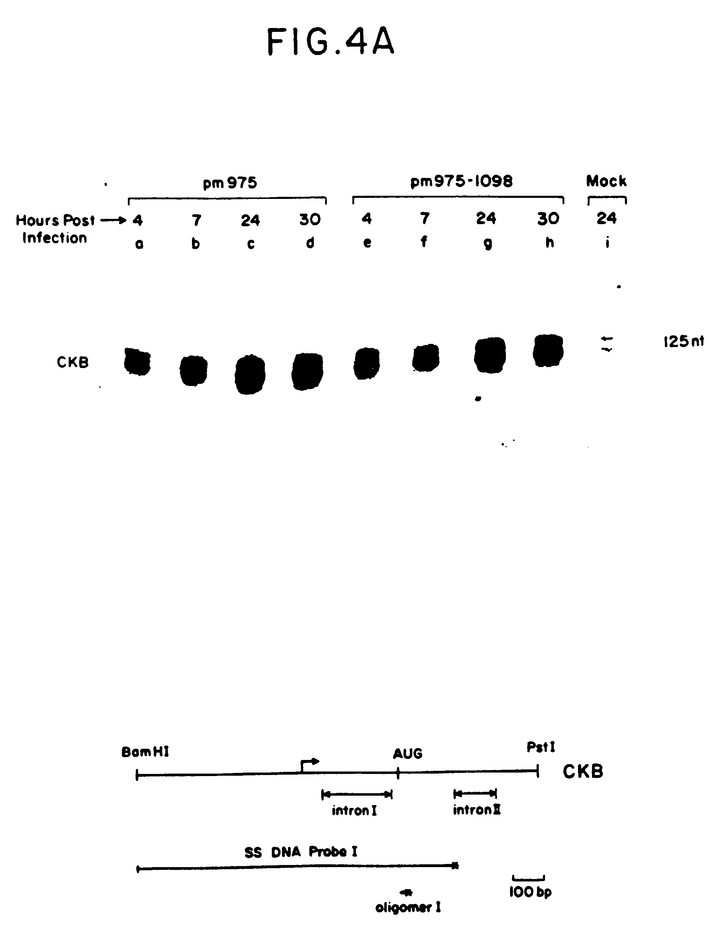

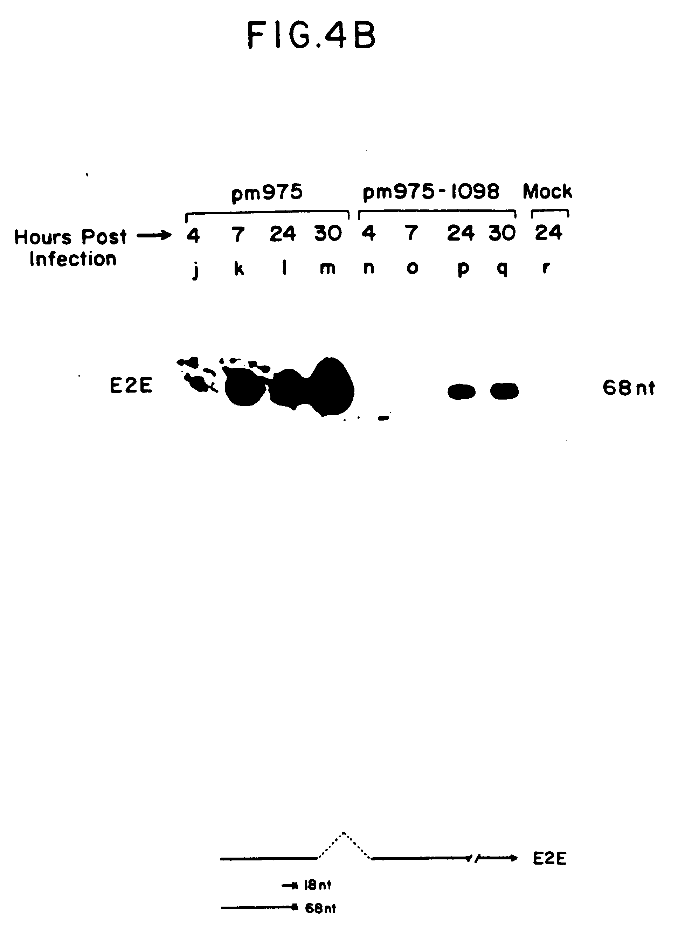

Figure 4 shows autoradiograms showing the results of hybridization assays of total cellular RNA harvested from HeLa cells infected with pm975 wild-type Ad5 virus, HeLa cells infected with the pm975.l098 mutant which has a point mutation in domain 3 or mock infected HeLa cells, using a probe specific for CKB or a probe specific for E2E. Panel A shows induction of CKB mRNA (lanes a-d, wild-type virus; lanes e-h, mutant virus; lane i, mock-infected or endogenous CKB level in HeLa cell lines used). Panel B shows the induction of E2E RNA (lanes j-m, wild-type virus; lanes n-q, mutant virus; lane r, mock-infected, showing no endogenous E₂E RNA. Panel C shows results of measurements of creatine kinase activity when cells were infected with wild-type and mutant virus at 5 pfu/cell and 50 pfu/cell. O-CKB activity in mock-infected plates, ●- in wild-type virus infected plates and Δ - in plates infected with pm975-1098 mutant. Results demonstrate that a point mutation in domain 3 (pm975-1098) that is defective for induction of E2E can induce expression of CKB.

Figure 5 shows autoradiograms showing the results of hybridization assays of total cellular RNA harvested from HeLa cells infected as described in Example 2 at 20 pfu/cell with virus expressing the 12S(dl 1500) or 13S(pm975) products. Lanes a and b, mock infected; lanes c and d, dl 1500 infected; lanes e and f pm975 infected with a probe specific for CKB mRNA. Results show that the 12S product of E1a which lacks domain 3 activates expression of CKB.

Figure 6 shows autoradiograms showing the results of hybridization assays of RNA from HeLa cells infected with wild-type or mutant viruses, as described in Example 2 with probes specific for brain creatine kinase (A) and E2E (B): Lane a, mock-infected; lanes b and g, wild-type pm975 virus, lane c, mutant d1312 virus (deleted for the E1a products); lanes d, e, h, i, mutant viruses pm975-936 and -953 (point mutation in domain 2); lanes f, j, mutant virus pm975-1098 (point mutation in domain 3).

Figure 7 shows that amino terminal deletions of E1a which disrupt transformation also result in an inability to induce CKB expression.

Figure 8 shows colocalization of the E7 and E1a target sequences in the E2 promoter attached to the CAT gene.

Figure 9 shows the structural similarity between adenovirus E1a and papillomavirus HPV-16E7 transforming proteins.

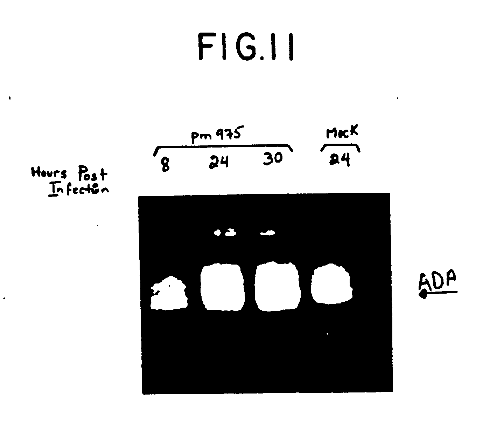

Figure 10 shows partial nucleotide sequences for adenosine deaminase and brain creatine kinase. An identical palindromic sequence, which is underlined and marked with an arrow, is shared by both enzymes.

Figure 11 shows autoradiograms showing the results of hybridization assays in which RNA harvested at 8, 24 and 30 hours post-infection from HeLa cells infected wild-type (pm975) virus (lanes a, b and c) or mock-infected (lane d) was probed for adenosine deaminase (ADA) production. Initial results show that ADA is induced by the E1a gene products.

Figure 12 shows part of the sequence of CKB gene, from which antisense oligonuclotides useful to block CKB translation can be determined.

### Detailed Description of the Invention

The present invention is based on the isolation, sequencing and characterization of a functional and complete copy of the gene for human brain creatine kinase (CKB). The creatine kinases are a family of enzymes which are involved in the maintenance of ATP at sites of cellular work. S.P. Bessman, Ann. Rev. Biochem., 54:831 (1985).

Cellular functions associated with the ability of viral oncogenes to transform cells have been identified. These involve energy and nucleotide pool level regulation by the creatine kinase shuttle system. Other pathways involved in adenosine metabolism, which regulate nucleotide levels locally in the cell, might involve other enzymes which can be regulated by the oncogene products. Such enzymes might have promoters whose sequence is similar to that of brain creatine kinase and adenosine deaminase.

As a result of these findings, it is now possible to disrupt the ability of a viral product (e.g., oncogene) or signals activated by those products to induce the expression of an enzyme (or enzymes) in a pathway involved in regulating cellular nucleotide levels and, thus, to reduce the effects of such activation or overexpression on cells. This can be carried out by interfering with the effect of the viral product on expression of the gene encoding the key cellular enzyme; by interfering with the activity of the activated or induced enzyme; by interfering with the activity of other components of the adenosine metabolic pathway, including adenosine uptake or the signals for their activation; or by interfering with any combination of the three.

Oncogenic products of DNA tumor viruses, particularly those DNA tumor viruses associated with transformation or tumors of epithelial cells, have been shown to have certain common functional, structural and sequence similarities which make it reasonable to expect that they have a common or similar effect on host cells which results in induction of cellular transformation. The creatine kinase system, which has now been shown to be linked to a DNA tumor virus, and transformation might be along a biochemical path which is modified by viruses.

In particular, it is known that the oncogenic products of two DNA viruses, the E7 protein of human papillomavirus (HPV) 16 and the adenovirus E1a protein, have many similar structural and functional roles in cells and are able to increase host gene expression. As described in detail herein, these similarities and the ability shown by these DNA viruses to induce cellular transformation appear to be linked to their ability to increase host gene expression, particularly their ability to increase expression of purine metabolic enzymes. In addition, the ability of these viruses to replicate has been shown to be linked to their ability to increase host gene expression of nucleotide metabolic enzymes.

The present invention relates to a method of inhibiting transformation in or metastasis of mammalian cells in which activity of one or more purine metabolic enzymes is elevated. This is carried out by inhibiting (reducing or eliminating) the ability of a DNA tumor virus, a factor produced by or characteristically associated with a DNA tumor virus (a DNA tumor virus factor) or other factor which has an effect corresponding to that of the DNA tumor virus or virus factor to induce cellular transformation or to maintain a transformed phenotype (DNA tumor virus factor equivalent), to increase purine metabolic enzyme activity. This is effected by interfering with the ability of the virus or factor to increase expression of such purine metabolic enzymes. It further relates to compounds or drugs useful in the present method.

In particular, through use of the present method, it is possible to inhibit the ability of a DNA tumor virus or a DNA tumor virus factor or its equivalent to increase the expression of host cell enzymes, such as creatine kinase, adenylate kinase adenylate cyclase and adenosine kinase, which participate in purine metabolism or which interact with these host cell enzymes. It is also possible by the use of inhibitors for these enzymes to counterbalance the induction effect caused by the virus. Thus, it is possible to inhibit the ability of the DNA tumor virus or virus factor to induce cellular transformation or to maintain the transformed state by preventing the induction or by chemically neutralizing it. As a result, cellular transformation, maintenance of transformation or metastasis is inhibited (i.e., it is prevented or occurs to a lesser extent than would be the case if the present method were not carried out or is reversed, wholly or in part, once a cell has been transformed).

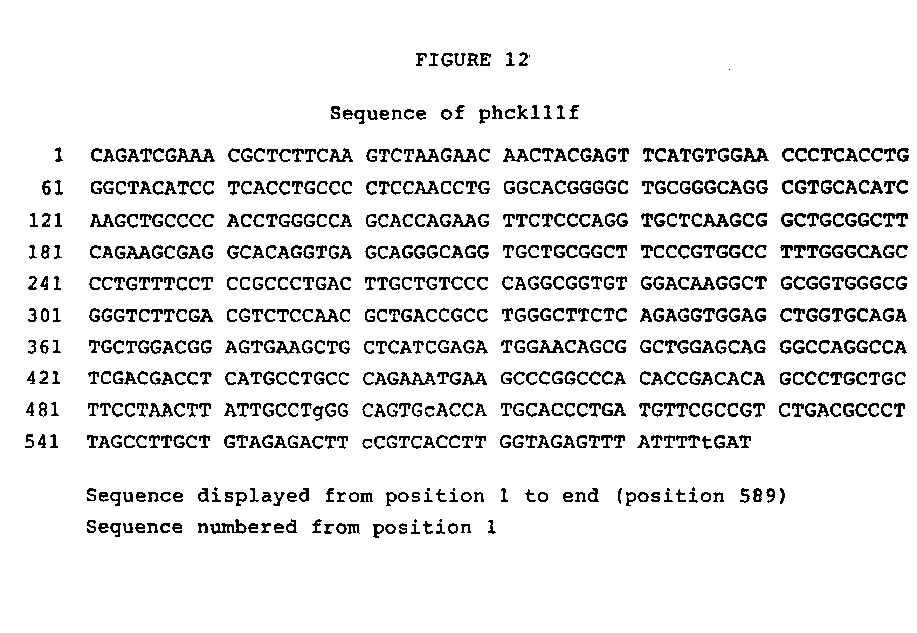

The present method of inhibiting transformation of mammalian cells in which purine metabolic enzyme activity is increased (i.e., greater than purine metabolic enzyme activity in untransformed cells) can have its effect by several different mechanisms. That is, the ability of a DNA tumor virus, DNA tumor virus factor or other factor to induce transformation can be interfered with by:
1) inhibiting the purine metabolic enzyme(s) whose activity in transformed cells is elevated as a result of the effect of the virus or factor. This can be carried out, for example, by administering a compound or drug which reduces the activity of the purine metabolic enzyme(s) or reduces the association of two or more purine metabolic enzymes;
2) decreasing the message level/preventing translation of the mRNA. This can be carried out by administering antisense constructs which are oligonucleotides selected to bind to a region of the cellular purine metabolic enzyme mRNA necessary for translation. For CKB, for example, this region is defined and is that region at the 3' end, shown in Figure 12;
3) interfering with transcription factors. This can be carried out by modifying the association of unique factors with their specific promoters. Drugs can be designed to prevent or modify the association and hence control the extent of initiation and hence products expressed from indicated adenosine genes;
4) by inhibiting interaction of viral products with host cell genes encoding the purine metabolic enzymes. This can be carried out by identifying the target and preventing the association and triggering of the promoter. The unique TTAA element on the CKB promoter, which is shared with the adenovirus E2E promoter and the ADA gene, might be an excellent candidate; and
5) by inhibiting interaction of viral product(s) with host cell product(s), the interaction of which regulates the activity or expression of purine metabolic enzymes. This can be carried out by compounds designed to bind to the viral products and inhibit binding of the host cell product with the viral product.

Because it is known that the activity of purine metabolic enzymes, such as creatine kinase is elevated in mammalian cells transformed by a DNA tumor virus or virus factor, and because there are enzymes which work in conjunction with it, such as adenosine kinase, adenylate kinase and adenylate cyclase, such enzymes might be useful as markers for identifying transformed cells.
For example, the occurrence (presence/absence or quantity) of one or more of these enzymes can be determined, using known enzyme assays or specific probes from the isolated genes. This can be an indicator or marker for detecting infection with virus or a transformation process occurring in cells and the information used for diagnostic purposes or for monitoring treatment in an individual in whom diagnosis has been made.

The present method of inhibiting cellular transformation can be carried out using existing drugs, analogues of such drugs, or drugs specifically designed for the purpose. Drugs can be administered to produce inhibition of cellular transformation by direct interaction with the virus (e.g., by inhibiting production of viral DNA or mRNA expression of the encoded protein); by blocking the domain or sequence of the virus which is responsible for inducing expression of a cellular gene involved in cell growth perturbing functions; by blocking the activity of a factor, produced by or characteristic of the DNA tumor virus, which is the inducer of such a cellular gene; or by acting directly upon host cell products which interact with a viral product to cause transformation (e.g., by inhibiting the enzyme activity of the activated enzymes). Drug analogues and drugs designed for the specific purpose intended are also the subject of the present invention.

The following is a description of DNA tumor viruses able to induce cellular transformation, which can be inhibited by the present method; of individual enzymes involved in purine metabolism and their interactions with one another and other cell constituents of the basis on which this inhibition is effected; of the use of the present method in inhibiting cellular transformation and of compositions useful in the present method. It is important to note that in each of the instances described, in which cellular transformation occurs, the activity of host cell creatine kinase is increased (greater than activity which occurs in the absence of transformation). Therefore, it is reasonable to expect that the present method can be used in the treatment of any tumor in which the activity of creatine kinase is increased significantly above activity in normal cells. Similarly, it is reasonable to expect that the present method can be used in inhibiting transformation of cells in which activity of other purine metabolic enzymes, particularly those which associate functionally with CKB, is increased.

It is also possible to inhibit metastasis of transformed cells (reduce the extent to which it occurs or prevent its occurrence) using the method and compounds described.

### DNA Tumor Viruses

### Huxan Papillomaviruses and Association with Human Angogenital Cancers

Recent investigation has established a strong association between certain human papillomaviruses (HPVs) and some types of human angogenital cancers. More than a dozen different HPV types have been isolated from epithelial tumors of the genital region. Precancerous lesions, such as moderate to severe cervical dysplasia and carcinoma in situ, as well as invasive cervical carcinoma, have been associated with HPV types 16, 18, 31 and 33 (Zur Hausen and Schneider, 1987 In: Howley PM Salzman N Eds. The Papovaviridae: The Papillomaviruses, Plenum, NY pp. 24-263). Of the HPVs which have been associated with anigenital malignancies, HPV-16 has been detected most frequently (greater than 60%) in biopsies from cervical carcinoma. RNA analyses for cervical carcinoma tissues and derived cell lines have revealed that the E6 and E7 ORFs are generally expressed, suggesting that their gene products may be necessary for the maintenance of the malignant phenotype. (Schneider-Gadicke and Schwarz, (1986) EMBO, 5: 2285-2292; Smolkin and Wett Stein (1986) PNAS, 83:4680.4684; Baker, C.C. et al., (1987) J. Virol, 61:962.971; Takebe, N. et al., (1987) Biochem Biophys Res. Commun. , 143:837.844). Morphological transformation of established rodent cells has been described for HPV-16 (Yasumoto, B. et al., (1986) J. Virol, 57:572-577; Tsunokawa, Y. et al., (1986) PNAs, 83:220.2203; Kanda, T. et al., (1987) Jpn J Cancer Res., 78:103.108). This transforming activity has been localized to the E6/E7 region (Bedell, M.A. et al., (1987) J. Virol, 61:3635.3640; Kanda et al., (1988) ibid). In addition, the HPV-16 E6/E7 region has been shown to encode a function capable of cooperating with an activated ras oncogene in the transformation of rat embryo fibroblasts. (Matlashewski, G. et al., (1987) EMBO, 6:1741.1746).

The immortalization function of HPV 16 has been mapped to the E7 ORF by Phelps and co-workers. (Phelps, W.C. et al., Current Topics in Microbiological and Immunology, 144 Springer Verlag Berlin Heidelberg, (1989). These researchers also found the E7 product of HPV-16 to have activities similar to those of the oncogenic product of another virus, the E1a protein of adenovirus.

### Similarities Between Human Papillomavirus E7 Onco-genic Product and Adenovirus E1a Oncogenic Product

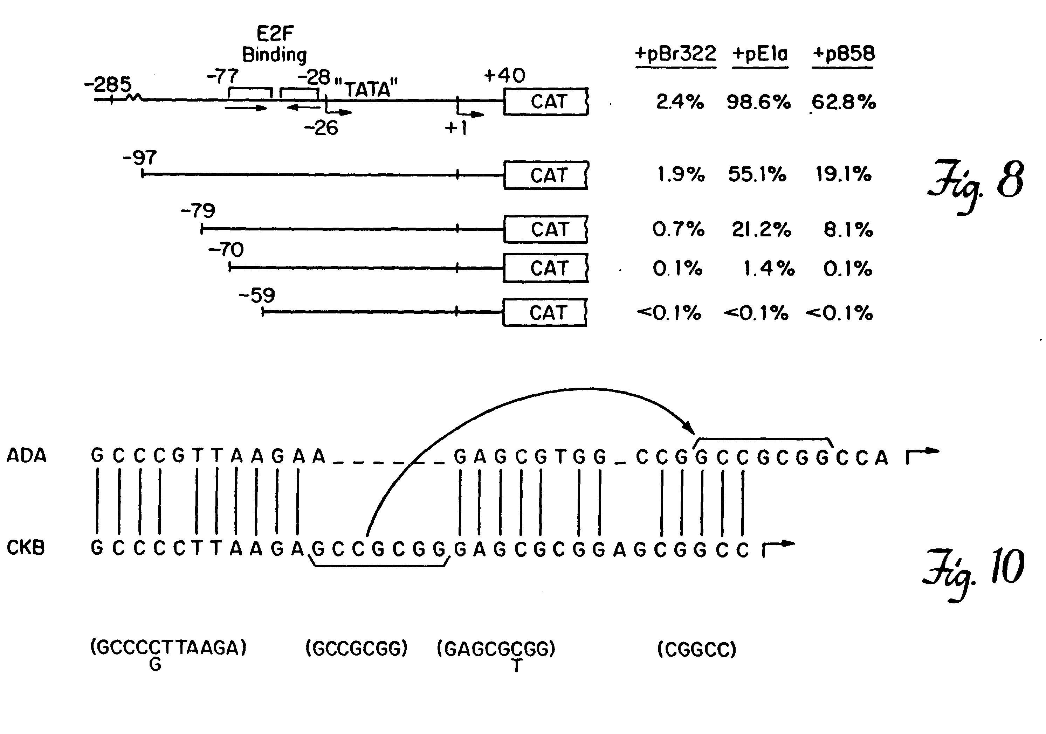

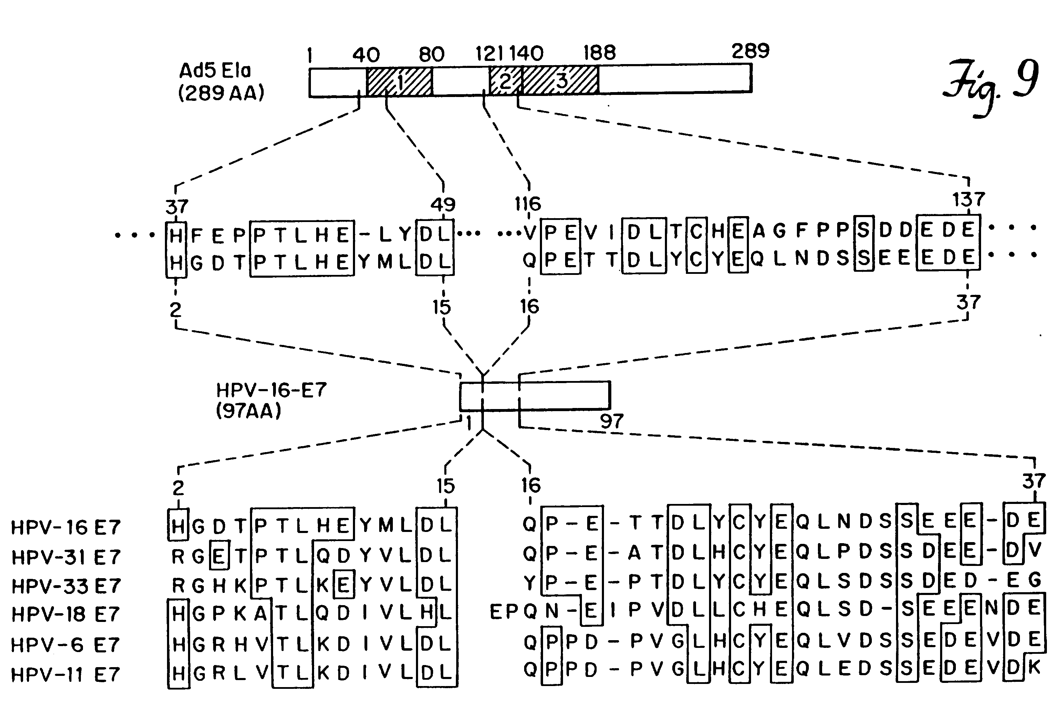

In summary, the papillomavirus oncogenic product and the adenovirus oncogenic product described above have been shown to have the following similarities:
1. Phelps, et al., has shown that E7 has transcriptional transactivation properties analogous to those of adenovirus E1a. That is, E7 can affect heterologous promoters, including the adenovirus E2 promoter. The adenovirus E2 promoter sequences required for E1a stimulation and HPV-16 E7 activation have been shown to be coincident. Figure 8 taken from Phelps, W.C., et al., Current Topics in Microbiology and Immunology: Transforming Proteins of DNA Tumor Viruses. Edited by Knippers and Levine, Springer-Verlag, pp. 153-166.
   a) The figure is a demonstration of localization of the E7 target sequence in the Adenovirus E2 promoter hooked to the CAT gene. Ba131 deletions with end points at -97, -79, -70 and -59 derived from the -285 to +40 AdE2CAT plasmid were generated. Five micorgrams of each Ad E2CAT plasmid together with 5 µg of either PBR322, PE1A (a plasmid encoding adenovirus E1a product) or p858 (a construct expressing the papillomavirus E7 product) were cotransfected into CV1 monkey cells and assayed for CAT actaivity. The locations of the E2F binding sites and its major and minor RNA initiation sites are shown.
2. Comparison of the amino acid sequences of HPV.16 E7 and adenovirus E1a proteins reveals regions of significant amino acid similarity which are well conserved within the E7 proteins of other papillomaviruses present in genital tissues. Figure 9 taken from Phelps, et al., (ibid).
   b) Figure 9 shows structural similarity between Adenovirus E1a and HPV-16 E7. A schematic diagram of the Ad5 E1a region is shown at the top of the figure with the conserved amino acid domains 1.3. Homologous (boxed) and is functional (stippled) amino acids of domains 1 and 2 from E1a which are found in HPV-16 E7 are located within the N-terminal 37 residues. The predicted amino acid sequences for this region of the E7 proteins of the other genital-associated HPVs are shown.
3. The conserved sequences between E1a and E7 mentioned above are restricted to domains 1 and 2 of E1a. Those sequences are important for the ability of E1a to transform and induce DNA synthesis. (Lillie, J.W. et al., (1986) Cell, 46:1043.1051).
4. Both E1a and E7 associate with the antioncogene product of the retinoblastoma (Rb) gene (N. Dyson et al., Science, 243:934-937 (1989)). The conserved sequences between the two proteins which are important for transformation are also important for the association with the Rb protein. This suggests that the two proteins interact with the same cellular metabolic events to cause transformation.
5. E7 can compliment the ras oncogene in transforming baby rat kidney cells in a similar fashion to E1a complementation (Phelps et al., ibid).

Thus, two DNA tumor viruses, each belonging to a different class of DNA viruses, have been shown to have structural and sequence similarities and to modulate host gene expression. Both E7 and E1a appear to possess multifunctions and it is reasonable to assume that their ability to modulate host gene expression is an important event in their ability to induce cellular transformation. If the cellular gene products which are targets for such oncogenic regulators which require the transforming domains of these DNA tumor viruses were identified, it would not only provide great insight into metabolic events which are affected during transformations, but also provide a means by which transformation by such DNA tumor viruses can be inhibited. In addition, it would make it possible to design markers useful in diagnosing infection with the responsible DNA virus and in monitoring subsequent transformation. It would also make it possible to design compounds or drugs useful to inhibit such activated events and serve as chemotherapeutic and antiviral compounds. As described in the following sections, it has been determined that at least one cellular enzyme which is involved in energy metabolism and purine pathways (i.e., creatine kinase) and which is a tumor marker is a reasonable target of DNA tumor viruses. The oncogene proteins of the DNA tumor viruses probably act on host/cellular genes and modify expression of the kinase gene, resulting in induction of a transformed state.

### Enzymes Involved in Energy and Purine Metabolism and Their Interactions

The brain isozyme of creatine kinase (CKB) has many features which are of relevance to the work described herein. Elevated levels of the CKB have been detected in tumor samples obtained from patients with small cell lung cancer (SCLS). Gazdar, A.F. et al., Cancer Res., 41:2773-2777 (1981). Marked elevation of CKB (as measured with a radioimmunoassay), was noted in 49 out of 50 cells lines established from patients with SCLC. Carney, D.N. et al., Cancer Res., 45:2913-2923 (1985). In contrast, CKB levels in non-SCLC lines were considerably lower, or negligible in most cases. The level of this enzyme is regarded as a reliable marker of SCLC and may be of functional importance in determining aspects of the small cell phenotype, including the neuroendocrine features unique to this tumor. Gazdar, A.F. et al., In: The Endocrine Lune in Health and Disease, (Becker, K.L. and A.F. Gazdar, eds.), W.B. Saunders & Co., Philadelphia, PA, pp. 501-1108 (1984).

Elevated levels of CKB in both tumor cells and patient serum have been associated with a wide range of human tumors. Examples are prostatic carcinoma, breast carcinoma, cancers of the ovary, uterus, cervix, bladder and anal canal. Rubery, E.D. et al., Eur. J. Canc. Clin. Oncol., 18:951.956 (1982); DeLuca, M. et al., Biochem. Biophys. Res. Comm., 99:189.195 (1981); Feld, R.D. and D.L. Witte, Clin. Chem., 23:1930-1932 (1977); Thompson, R.J. et al., Lancet, ii:673-675 (1980). In many cases, there is a good correlation between the progression of metastasis and levels of CKB in serum. Zweig, M.H. et al., Clin. Chem., 25:1190-1191 (1979). In many patients the levels of CKB in serum seem to respond to clinical treatment of the tumors. Thompson, R.J. et al., Lancet, ii:673-675 (1980).

The B isoenzyme is under hormonal control. It is known to be induced by estrogen in the female genital tract. Malnick, S.D.H. et al., Endocrinology, 113:1907.1909 (1983). Its induction is rapid (within one hour) and it is identical to the previously identified estrogen induced protein. It has subsequently been found to be stimulated by vitamin D metabolites in cartilage, (Somjen, D. et al., FESS Letters, 167:281.285 (1984)), bone and kidney (Somjen, D. et al., Biochem. J., 219:1033.1039 (1984)), as well as by PTH and prostaglandin E2 in bone cells in culture (Somjen, D. et al., Biochem. J., 225:591-595 (1985)). Golander, A. et al., Endocrinology, 118:1966.1970 (1986) showed that human growth hormone (hGH) induces CKB expression in kidney, liver and epiphysis, both in vivo and in vitro. Its induction by hGH is followed by a parallel increase in DNA synthesis.

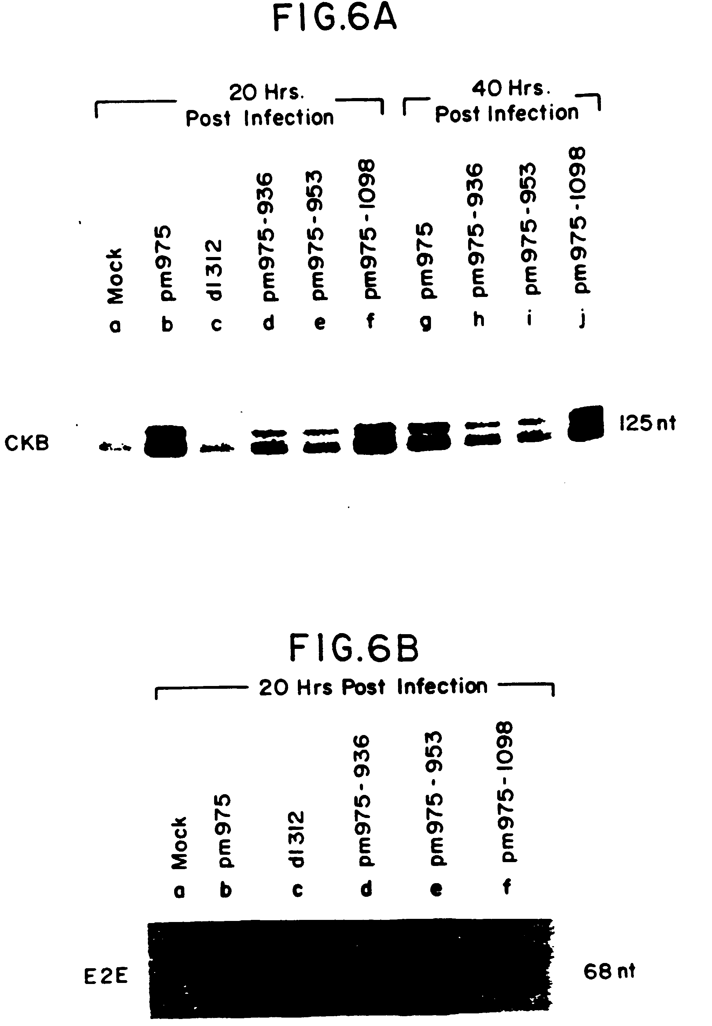

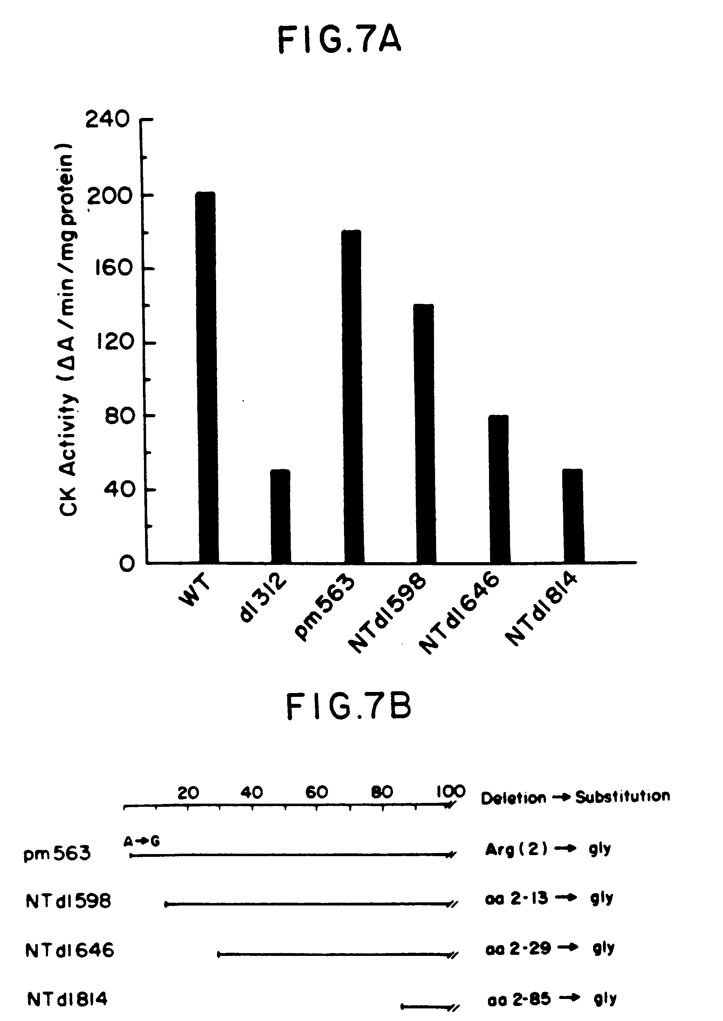

It has now been demonstrated that the promoter of the human CKB gene has a strong sequence similarity to the adenovirus E2 promoter, Figure 3B. Further, it has been demonstrated that both brain creatine kinase and its mRNA levels are induced by the oncogenic products of the E1a gene of adenovirus type 5, Figures 4-7. In particular, through the use of adenovirus mutants in which a domain of the E1a region is nonfunctional, it has been shown that transforming domain 2 of E1a is required for viral induction of CKB, Figure 6. Also, the amino terminus and domain one of E1a, which are involved with transformation and induction of DNA synthesis are needed for CKB induction, Figure 7. Thus, it has now been demonstrated that the oncogenic products of a DNA virus induce the expression of a cellular enzyme (CKB) which not only has a key role in cellular energy metabolism, but also is known to be present at elevated levels in a variety of tumor cells. The induction correlates with the ability of E1a to induce DNA synthesis and to transform, implying that it is essential for E1a and other related cellular analogues to express these functions.

It is significant that the nucleotide sequence of the promoter region of adenosine deaminase (ADA) closely resembles the promoter region of the brain creatine kinase gene with a palindromic sequence being shuffled. A possible coregulation (up or down regulation for one or both) must be an important step in viral invasion and transformation. This is of particular interest because, like CKB, adenosine deaminase participates in adenosine metabolism (and, thus, DNA synthesis, replication and 2nd messenger production). Suppression or deletion of adenosine deaminase in children is associated with severe immunosuppression. Hirschhorn, R., Ciba Found. Sym., 68:35.54 (1978). Chemical inhibitors of adenosine deaminase have been shown to suppress replication of a wide variety of viruses in their host cells. T.W. North and S.S. Cohen, Proc. Natl. Acad. Sci., USA, 75:4684-4688 (1978). This suggests that the enzyme is important for replication of many viruses or is involved with a signalling pathway affected by viral products of their cellular analogues. Like the association of CKB with SCLC, adenosine deaminase is associated with T cell tumors, and inhibitors of ADA have been used in the treatment of hairy-cell leukemia (J.B. Johnston et al., Cancer Research, 46:2179-2184 (1986)). Patients with AIDS have elevated servm levels of ADA but undetermined levels intra-cellularly. M.J. Cowan et al., Proc. Natl. Acad. Sci., USA, 83:1089.1091 (1986).

CKB levels have been shown to be elevated in tumors, and have now been demonstrated to be inducible by adenovirus. The induction by adenovirus is closely associated with the transforming ability of the oncogenic products of the virus, suggesting that transformation by adenovirus requires the expression of CKB. The striking similarity between the regulatory regions of CKB, adenosine deaminase and adenovirus E₂E, suggests that the two cellular genes are part of a metabolic pathway that is affected by signals from viruses to replicate and signals involved in oncogenic transformation. We know that both of these cellular genes are involved in adenosine metabolism, and with the synthesis of DNA nucleotide precursors, and hence are tightly linked to replication.

Oncogenes and anti-oncogenes that are capable of inducing growth changes in the cell and inducing a transformed phenotype are now thought to act as elements of a signalling pathway that allows cells to respond to environmental signals. It has been suggested that nuclear oncogenes are capable of inducing the expression (transactivation) of key growth related genes in the nucleus. However, until the present time, there has been no solid evidence to support this hypothesis. The E1a oncogene has presented a dilema for investigators studying transformation because although the E1a oncogene can transactivate viral and a few cellular genes, this ability seems to be distinct from its ability to transform cells.

For the first time, it has been shown that a product of the adenovirus E1a region turns on the expression of a cellular gene that is at the heart of energy metabolism or production for the cell, that is involved in regulation of energy and nucleotide pools, and that is expressed at high levels in a wide range of tumors. It has been shown that induction corresponds with the transforming ability of the molecule, indicating that brain creatine kinase is on a signal pathway that is required for E1a to function as an oncogene.

Another important cellular gene, which encodes adenosine deaminase, has been shown to have a striking resemblance (sequence similarity) in the promoter region to brain creatine kinase. It also appears, although from preliminary data, that the adenosine deaminase promoter is regulated by the E1a products (Figure 11). This is of particular interest because adenosine deaminase, like brain creatine kinase, is a tumor antigen (a thymus leukemia antigen), and is also involved in adenosine metabolism. It has been shown to be up or down regulated by other viruses. Evidence demonstrates that the two branches of adenosine metabolism (i.e., the kinase and the deaminase) are affected by viral infection and changed in tumor metabolism. The presence of these regulatory sequences in replication genes of tumor viruses suggests a site of communication between the virus and the host cell. Adenosine metabolism and production of a second messenger, cyclic adenosine monosphosphate (cAMP) are part of the link.

There is evidence that suggests that CKB and ADA are members of a larger metabolic system in which the promoters of participating enzymes bear sequence similarities to one another, end are normally regulated in a coordinated manner, but that there is a second level of regulation imposed by viral products. The viral products may act on members of this metabolic pathway either directly (e.g., by binding to cellular products, such as the retinoblastoma gene product, which alters gene expression) or indirectly (e.g., by altering the function or action of genes or gene products involved in a cascade of events leading to altered expression of genes in this metabolic system). It is reasonable to suggest that in acting as an oncogene, E1a turns on or activates these cellular genes.

Furthermore, at least a fraction of both ADA and CKB has been shown to be membrane-associated. The concentration of membrane-associated adenosine deaminase binding protein has been shown to vary in different tumors. This protein seems to anchor the adenosine deaminase to the membrane, to modify its activity, and to carry adenosine to other parts of the cell, including the nucleus. A subpopulation of CKB is bound to the inner membrane as well. Another enzyme involved in adenosine metabolism is membrane-bound adenylate cyclase and adenylate kinase. Adenylate kinase associates physically with creatine kinase. It is reasonable to predict that a complex is formed in the membrane around the adenosine receptor. A signal that is generated from a virus, or is the inducer of a transformation process, might be introduced into the cell by the adenosine receptor or the adenosine binding protein or the complex. This signal is translated into modification of adenosine metabolism leading to local adjustment of nucleotide pools (ATP and GTP). Secondary messengers are thought to be activated secondary to the activation of this cascade of enzymatic reactions. Cyclic AMP (cAMP) is a product of adenosine metabolism and could be a secondary mediator. The present invention, thus, relates to interference with viral or oncogenic induction of the expression or activity of any member of this membrane-associated complex.

It is also possible that viral products which activate some cellular genes activate a subpopulation of lipids associated with the membrane, which are then released as secondary messengers to the nucleus as inducers of replication. Most of these lipids are activated by phosphorylation by ATP and CKB might be involved indirectly in activating these lipids. Viral products might also release growth factors that act on the membrane-associated complex and might also activate ion channels in the membrane or hormone receptors. The activation of such a cascade, and the local adjustment of nucleotide pools, resulting from induced levels of CKB or ADA, might trigger G-binding proteins (e.g., p21), adding another dimension to the signal transduction.

Another sub-branch of the pathways related to adenosine metabolism is the generation of S-adenosine methionine, which is involved in DNA methylation and which could be coregulated.

It is probable that viral products interact with some of the above mentioned receptors or membrane components. For example, HIV virus is associated with changes in adenosine deaminase in T cells, and the HIV early gene product, TAT, has been demonstrated to be secreted and taken up by cells. The above mentioned receptors, adenosine, or adenosine binding proteins or the complex of proteins, might be mediators for this uptake.

The anti-oncogene Rb (retinoblastoma) gene product has been shown to interact with domain: 1 & 2 in the E1a protein, SV40 large T antigen and papilloma virus HPV-16E7 product. Thus, it is possible that these oncogenes work indirectly (i.e., their effects might be mediated by the Rb gene product, if Rb is a master down regulator of proliferative genes). Other cellular proteins have been shown to interact directly with E1a, and might mediate some of its functions. A sequence present on the promoter region of the CKB gene which is also present on the promoter region of adenosine deaminase gene (Figure 8), might serve as the direct target for the Rb gene and those other cellular proteins.

### Identification of Cellular Gene Products Which are Targets for Oncogenic Regulators

It is reasonable to expect that a cellular gene product, creatine kinase, which is an enzyme involved in maintenance of ATP at sites of cellular work or energy production, is a target of oncogenic products of DNA tumor viruses, such as the HPV E7 product and the adenovirus E1a product, which act as regulators and modify or regulate expression of cellular genes. In addition to discrete DNA tumor viruses, there appear to be factors which are consistently present in or secreted from some transformed cells or tumor cells, which in and of themselves are capable of inducing DNA synthesis in host cells and probably are able to modulate cellular gene expression with much the same effect that a DNA tumor virus has (i.e., increase in expression of the cellular gene). Such factors are referred to herein as DNA tumor virus factors.

As discussed below, creatine kinase has several characteristics which support the idea that it is such a target of oncogenic products of DNA tumor viruses. As is also discussed below, other enzymes which have a similar role in cellular metabolism (e.g., purine metabolism and nucleotide level regulation) and which work in association with CKB are likely to be targets of oncogenic products as Well. Thus, interference with the ability of an oncogenic product of a DNA tumor virus or DNA tumor virus factor to regulate expression of enzymes which participate in purine metabolism and/or interference with another enzyme(s) which interacts with the purine metabolic pathway enzyme(s) will result in inhibition of or interference with the ability of such viruses or factors to transform cells. This should be particularly useful in inhibiting tumor formation, growth or metastasis in epithelial cells and, particularly, in epithelial cells of the cervix, in which HPV is known to be present. The method of the present invention makes it possible to inhibit the ability of DNA tumor viruses to act as mediators of cell transformation, and thus, to inhibit cell transformation or tumor formation or spread, by acting upon production of viral DNA, RNA or encoded oncogenic products, as well as by interfering with other viral or cellular products which normally interact or cooperate with viral or cellular products to bring about cell transformation.

It has now been demonstrated that CKB is regulated by E1a. Transforming domains one and two which are partially shared with other DNA tumor viruses are required for the induction observed.

The papilloma viruses (e.g., type 16) which are associated with malignancies in the cervix encode a protein (the E7-encoded protein) which is very similar in sequence and function to that of the E1a-encoded protein in the transforming region. In particular, several key similarities have been identified: 1) the E7-encoded protein has amino acid sequence homology to the E1a-encoded protein in the important transformation domain; 2) the E7-encoded protein, like the E1a-encoded protein can act to turn on the adenovirus E2 promoter; 3) both proteins interact with similar host proteins, such as the retinoblastoma gene product; and 4) the E7-encoded protein can replace the E1a-encoded protein in transformation assays in which complimentation with ras is tested.

It is also important to note that the sequence of the adenovirus E2 promoter, which controls the replication of adenovirus, has striking similarity to that of the cellular CKB promoter. Either E1a of adenovirus or E7 of papillomavirus virus can induce the E2 promoter and they both require the first 90 bases of the promoter for induction. E1A turns on CKB expression and because the first 90 bp of the CKB promoter sequence resemble those of the E2 promoter, it is reasonable to expect that E7 acts similarly (i.e., turns on CKB expression). In addition, the fact that the transformation domains 1 and 2 of E1a are required for CKB induction by the adenovirus and the fact that the same sequences of the two domains are partially conserved in the E7 protein of papillomavirus strongly suggest that CKB will be induced by E7 and would require the transformation domains of E7. Mutations which disrupt the ability of E1a to transform are disfunctional for CKB expression. These crucial amino acids are conserved in the transforming part of E7. It is likely that these two DNA tumor viruses cause some similar modifications in the host cell metabolism and hence work at least partially via common mechanisms to cause transformation. It has been shown that CKB associates with the ability of E1a to transform cells. It is reasonable, based on experimental proof and the literature, to predict that creatine kinase or the metabolic pathway is similarly affected in epithelial cells of the cervix which are infected by papillomaviruses associated with malignancies. After infection of the cervix by such a virus and expression by the virus of the encoded transforming proteins, increased expression of CKB and the enzymes working in conjunction with it (e.g., adenylate kinase and cyclase) occurs.

The following is a description of: 1) the isolation and characterization of a functional and complete copy of the human brain creatine kinase gene, particularly as to the strong sequence similarity between the brain creatine kinase gene promoter and that of the adenovirus E2E promoter; 2) activation of creatine kinase expression by the oncogenic products of the E1a region of adenovirus type 5; 3) the amino acids involved in E1a induction of CKB are conserved in many DNA tumor viruses suggesting that they all will induce the creatine kinase system; 4) means by which the oncogene product-creatine kinase gene interaction can be disrupted or counterbalanced; and 5) the fact that creatine kinase associates with other proteins indicates that these too will be an appropriate target for oncogenic product regulation.

### Isolation and Characterization of a Functional and Complete Copy of the Human Brain Creatine Kinase Gene

As described in detail in Example 1, a human genomic library was screened with a rabbit muscle-specific (M-) creatine kinase cDNA probe. Briefly, a 180-bp rabbit cDNA probe (M180) was prepared by primer extension of an M13 mp8 clone using [³²P]dATP as the radionucleotide and purified as described by Maniatis et al. Maniatis, T. et al., Molecular Cloning: A Laboratory Manual, Cold Spring Harbor Laboratory, Cold Spring Harbor, NY (1982). The 180bp probe was used to screen a human lymphocyte-derived genomic library in the Charon 4A lambda phage vector. Twenty-four potential positive clones were isolated and one clone (16B2), which gave the strongest hybridization signal, was chosen for further study. It was shown to contain a 12-kilobase pairs (Kbp) insert of human DNA.

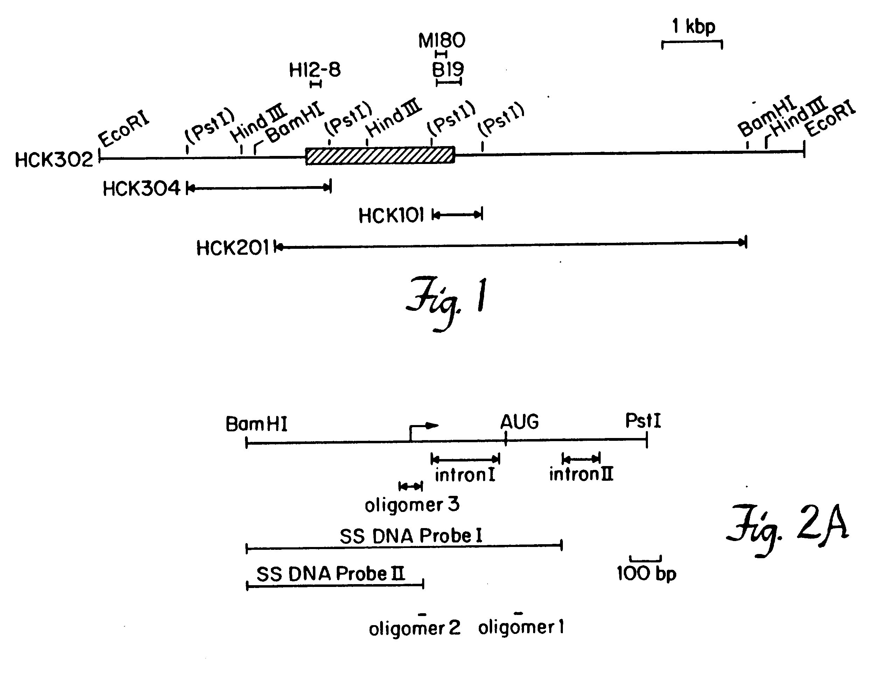

The 12Kbp insert was cloned into plasmid, pBR322, producing plasmid pHCK302 (Figure 1). The M180 probe hybridized to a 0.9-Kbp PstI fragment and shares with it 95% sequence homology. Rabbit B isozyme cDNA probes were used to -determine whether the clone encodes the remainder of the human creatine kinase. As described in Example 1, results showed that the entire coding sequence for human brain creatine kinase is encompassed by a region of 2.5Kbp that is embedded within the 12-Kbp genomic fragment isolated from the initial screen of the library. Plasmid pHCK302 was shown to express a function brain creatine kinase when transfected into BALB/c/3T3, LtK-and human HeLa cells. Subclone pHCK201, (Figure 1) which contained only 850 bp of 5'-flanking sequences, also expressed the enzyme to comparable levels.

The 5'-flanking region was analyzed, using a variety of synthetic oligonucleotides, to map the transcriptional start site (Figure 2A) and to identify the promoter element of the brain creatine kinase gene. This work is described in detail in Example 1 and results are presented in Figures 2 and 3. Results suggested that two promoter elements are present in the brain creatine kinase gene. Most promoters consist of a TATA box located approximately 30 bp upstream from the start site of transcription and additional upstream promoter elements are located between 30 and 100 bp upstream from the 5'-end. In vivo and in vitro transcription analysis and DNA binding studies have identified a few conserved elements which bind regulatory proteins. The gene for human creatine kinase B has two TA-rich sequences, a TTAA sequence that is centered at -26 from the presumptive transcription start site and a TATAAATA sequence that is centered at -62 (Figure 3A). There are two possible CAT sequences GCCAATG (-52) and GGCCAATG (-82), three copies of the Spl binding sequence GGGCGG occur at -46, -120, -144, and a GCACCACCC sequence at -98. Benoist, D. et al., Nucleic Acids Res., 8:127.142 (1980); Dynan, W.S, and R. Tjian, Cell, 42:559-572 (1985); Dynan, W.S. and R. Tjian, Cell, 32:669-680 (1983); Dynan, W.S. and R. Tjian, Nature, 316:774.778 (1985); Myers, R.M. et al., Science, 232:613-618 (1986). Because each CAT sequence is positioned just upstream of each TATA element, the sequence motif is suggestive of a compound of two promoter elements. Deletion and mutation work on these sequences should give insight on the role, if any, that these elements might have in vivo.

Because the TTAA sequence at -30 is located at the expected position for a TATA box, it is probably the functional element which initiates transcripts from the promoter. The presence of the exact TATA consensus sequence (TATAAATA) at position -62, which is preceded by a CAT (GGCCAATG) sequence at -82, raises the possibility that, under some conditions, this unit of the 5'-flanking sequence acts as a second promoter and that transcripts could initiate from a site different from what was mapped here. The prolactin gene's TA-rich sequence, which is distinct from the TATA box, can direct initiation of transcription under certain conditions. Barron, E.A. et al., New Insights Into the Regulation of Transcription, 6th Annual Stonybrook Symposium, p. 10, Stonybrook, NY (1987).

Results also showed that there is a remarkable similarity of the B isozyme promoter to the adenovirus E₂E (also designated "E2E") promoter region (Figure 3B). This region of the E₂E gene has two overlapping promoter elements that evidently direct expression from two transcriptional start sites. Four elements within this promoter (denoted regions I-IV in Figure 3B) have been identified as required for efficient expression. Zajchowski, D.A. et al., EMBO Journal, 4:1293-1300 (1985); Elkaim, R. et al., Nucleic Acids Res., 11:7105-7117 (1983). These four elements and a fifth region of strong sequence similarity are present in the 5'-region of the gene for the creatine kinase B isozyme. Because the E₂E promoter is inducible by the adenovirus EIa gene product, work was also carried out, as described below, to determine whether the EIa induces expression of the B isozyme.

### Assessment of Activation of Creatine Kinase Expression by the Oncogenic Products of the E1a Region of Adenovirus Type 5

The promoter region of the human brain creatine kinase gene, thus, has been shown to have strong sequence similarity to the adenovirus E2E promoter.

The similarity between the adenovirus E2E promoter and the promoter for human brain creatine kinase is restricted to promoter sequences required for wild-type levels of expression of E2E. As discussed in Examples 2-5 and Figures 4-7, it was demonstrated that expression from the brain creatine kinase promoter is regulated by E1a products. Initial data showed that mammalian cells (e.g., kidney cells) transformed by adenoviruses which expressed high constitutive levels of E1a also expressed high levels of brain creatine kinase. Transient co-transfection experiments in different mammalian cell lines in which brain creatine kinase was introduced on a plasmid, with or without an E1a expressing plasmid, showed increased (as much as 6 to 11.fold) activity in the presence of E1a. These results indicated that expression of brain creatine kinase is activated by all or a portion of the E1a gene product. It was subsequently demonstrated, as described in Examples 1 and Figures 2-5, that both enzyme and mRNA levels are induced by the oncogenic products of the E1a region of adenovirus type 5; that domain 3 is not necessary for activation of creatine kinase expression; and that transforming domains 1 and 2 are required for induction.

The E1a proteins of adenovirus are divided into three regions or domains. Induction of viral and cellular promoters requires domain 3 of E1a. However, activation of creatine kinase expression was shown to occur upon infection with adenovirus mutants that are defective in this domain. However, transforming domain 2, which is important for association of E1a with the retinoblastoma gene product (Rb), was shown to be required for induction. The polypeptide encoded by domain 2 is small (about 20 amino acids) and has been shown to be essential for the activity of E1a on cell growth. This difference in effect was demonstrated using adenovirus mutants with selected defects, as described in Examples 2-5.

The proteins encoded by the E1a region are necessary for efficient viral replication in permissive human cell lines and for viral transformation of nonpermissive rodent cells. The major proteins are nuclear phosphoproteins of 243 and 289 amino acids, which have the same amino and I carboxy termini and differ only by the presence of 46 additional amino acids in the 289 amino acid polypeptide. F.L. Graham, The Adenoviruses, Ginsberg, H. ed., Plenum Press, New York (1984). These are products of 12S and 13S RNA species, respectively. The E1a proteins contain three distinct domains that are strongly conserved among adenovirus subgroups and species. H. van Ormond, J. Maat, R. Dijkena, Gene 12, 63 (1980). D. Kimelman, J.S. Miller, D. Porter, B.E. Roberts, J. Virol, 53, 399 (1985). The vast majority of domain 3 is unique to the 289 amino acid protein and is important for trans-activation of early viral promoters. A.J. Berk, Ann. Rev. Genet. 20, 45 (1986). J.W. Lillie, M. Green, M.R. Green, Cell 46, 1043 (1986). J.W. Lillie, P.M. Loewenstein, M.R. Green, M. Green, Cell 50, 1091 (1987). Domains 1 and 2 are required for transcriptional repression, transformation, and induction of DNA synthesis, but not for activation of transcription. J.W. Lillie; M. Green, M.R. Green, Cell 46, 1043 (1986). J.W. Lillie, P.M. Loewenstein, M.R. Green, M. Green, Cell 50, 1091 (1987). E.K. Moran and M.B. Mathews, Cell 48, 177 (1987). E. Borrelli, R. Hen, P. Chambon, Nature 312, 608 (1984). A. Velcich and E. Ziff, Cell 40, 705 (1985). F.L. Graham, The Adenoviruses, Ginsberg, H. ed., Plenum Press, New York (1984).

A range of human cell lines were infected with wild-type adenovirus 5, and creatine kinase activity was monitored for up to 36 hours post-infection. Infections were carried out in the presence and absence of AraC, a compound which inhibits replication of the virus and thereby maintains a desired level of E1a. HeLa cells, U937 (MIT Tissue Culture Collection), and an established transformed SCLC line (NCI-H69) showed a strong induction (3-12 fold) of creatine kinase activity by 16-24 hours post-infection. In contrast, infecting the same types of cells at high multiplicities with the deletion mutant adenovirus d1312, a mutation that obliterates the E1a region, showed no effect on brain creatine kinase expression.

Domain 3 of the E1a gene was shown to be unnecessary for induction of brain creatine kinase (Example 2). A Gly- Arg point mutation in domain 3 (mutant pm975-l098) impaired E1a-activation of E2E and of other early viral promoters. J.W. Lillie et al., Cell, 46:1043 (1986); J.W. Lillie et al., Cell, 50:1091 (1986). However, this mutation had no effect on the ability of E1a to activate expression of creatine kinase in HeLa cells at multiplicities of infection that range between 5 and 50 pfu/ cell. By primer extension of end-labeled oligonucleotides, brain creatine kinase and E2E mRNAs were analyzed. The induction of brain creatine kinase mRNA synthesis by wild-type adenovirus pm975 (Figure 4, lanes a-d) and mutant adenovirus pm975-1098 (Figure 4, lanes e-h) contrasts with the behavior of E₂E RNA synthesis where the pm975-1098 mutation prevents E1a-activation of E2E RNA synthesis, as shown in (Figure 4B, lanes j-m (wild-type) and lanes n-q (mutant). Figure 4C shows that creatine kinase activity is induced to similar levels upon infection with both the wild-type and 1098 mutant adenovirus, at multiplicities of infection of about 5 and 50 pfu/cell.

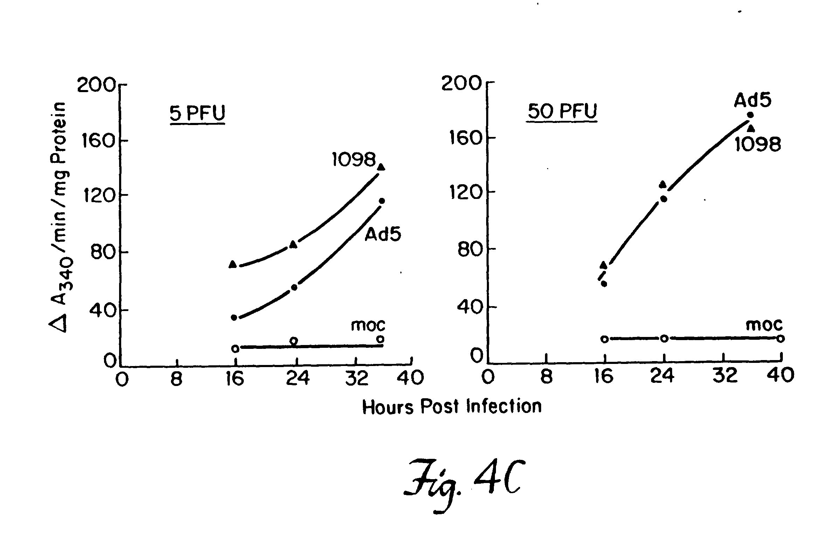

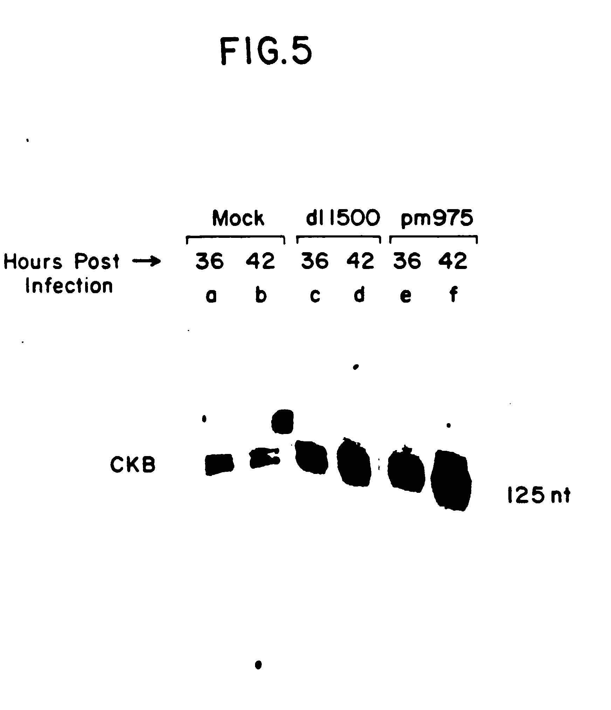

Infection with the d11500 adenovirus, which expresses the E1a 12S product lacking domain 3, and results in synthesis of the 243 amino acid polypeptide, also resulted in induction of CKB expression (Figure 5). Compared with mock-infected cells (lanes a, b), significant induction of CKB was observed in d11500 infected plates (lanes c,d). The induction was slightly lower than that seen with wild-type (lanes e, f) and requires a longer period (which may be a result of a low level of expression of the 243 amino acid E1a product). Mutant adenovirus d1312, which lacks the E1a region, had no effect on CKB expression, even at long time points. Collectively, the results shown in Figures 4 and 5 demonstrate that domain 3 of E1a is not essential for induction of CKB expression.

HeLa cells were infected with the wild-type virus or with one of two mutant viruses which have point mutations in domain 2, as described in Example 4 and Figure 6. These two point mutations (pm975-936(Glu126.Gly) and pm975-953 (Ser132-Gly) render the E1a 289 amino acid gene product defective for collaboration with the activated ras oncogene in transforming primary rat kidney cells. J.W. Lillie et al., Cell, 46:1043 (1986). The mutants are also poor inducers of DNA synthesis in growth arrested cells, but have no effect on the induction of the early viral genes. J.W. Lillie et al., Cell, 50:1091 (1987). The induction of brain creatine kinase mRNA was severely impaired in plates that were infected with either of the domain 2 mutant viruses. (Figure 6A, lanes d, e). The mock infection (i.e., without virus) (lane a) and infection with the d1312 deletion mutant (lane c) show the same level of CKB which corresponds to the endogenous amount in these particular cells. Infection with the wild-type and domain 3 mutant (pm975-1098) virus gave the expected induction (compare lanes b and f). The same observations were made when RNA was harvested at 40 hours post infection (Figure 6A, lanes g-j).

Figure 6B shows the results of probing of the same RNA samples for the E2E product with a hybridization probe specific for the E2E protein. The results revealed that both domain 2-point mutants activated expression of E2E to a level similar to that of the wild-type virus (compare Figure 6B, lanes d and e, with b), while the domain 3-deleted point mutant (pm975-1098) and E1a deletion mutant (d1312), were both defective for activation (Figure 6B, lanes f and c). These results distinguish the parts of E1a that activate expression of CKB from those which activate E2E.

Further experiments investigated the effects of domain 1 mutations on brain creatine kinase expression.

Figure 7 shows that amino terminal detections of E1a into domain 1 obliterate the induced expression of CKB. HeLa cells were infected with amino terminal deleted viral constructs. Panel a shows creatine kinase actually represented by bars. Panel b shows amino acids deleted into each mutant and names given by Whyte and Co-wokers. Whyte P. et al., J. Virol., 62:257-265 (1988). The same observation is seen when RNA harvested from cells is probed with CKB specific probes (data not shown). Hence the induction of CKB a major enzyme in energy metabolism and nucleotide regulation associates with the ability of E1a to transform. It leads to this conclusion that it must be part of a mechanism by which cells are transformed by some DNA tumor viruses or their equivalents.

### Compositions Useful in the Present Method of Inhibiting Purine Metabolic Enzymes

Compounds which inhibit CKB and/or other enzymes which participate in purine metabolism, directly or indirectly (i.e., by interacting with enzymes in the pathway) will have great value in preventing and/or treating cervical tumors and other tumors characterized by high levels of these enzymes. These compounds can be transition state analog covalent inhibitors applied locally in the' cervix. In addition, inhibitors of the enzymes that work in conjunction with CKB, can now be designed and used, in order to make control of the effect on CKB tighter. For example, inhibitors of adenylate kinase, an enzyme that physically and functionally associates with creatine kinase, can be designed. Such compounds, alone or in combination (in combination they control ATP levels more tightly), are useful as antiviral, antitumor compounds in the cervix.

### Inhibitors of Creatine Kinase

### Potential Drugs (antitumor, antiviral)

The following are compounds or drugs which can be used, alone or in combination, in the method of the present invention in order to inhibit the purine metabolic enzyme indicated. It is to be understood that these are just examples of compounds which can be used and that this is not intended to be limiting in any way. These compounds, modifications of these compounds, and new compounds can also be used.

### Examples of Compounds That Inhibit Creatine Kinase

1. A multi-substrate analogue, such as a disubstrate compound having both the creatine entity and the nucleotides ADP or ATP, covalently linked

| | | |
|---|---|---|
| a) | Creatine-ADP | Creatine-ATP |
| b) | Creatine-R-ADP | Creatine-R-ATP |
| | where R is a spacer, such as(CH₂)ₙ | |
| c) | Creatine-R-ADP with methylene groups replacing some or all of the phosphorus back-bone, to facilitate uptake by cells | Creatine-R-ATP with methylene groups replacing some or all of the phosphorus back-bone, to facilitate uptake by cells |
| d) | Variations of Creatine-R-ADP | Creatine-R-ATP |

which facilitate uptake of molecules by cells, by reducing the net charge on the molecules
2. Modifications of compounds generated by Kenyon and co-workers, which inhibit creatine kinase; appropriate modifications enhance uptake by cells or make it specific by attaching it to ADP or its metylene derivatives
Examples of such compounds are:
a) 1-carboxymethyl-2-iminoimidazolidine-4-one 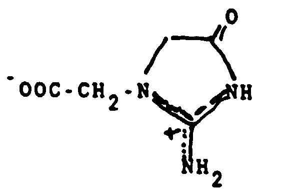
b) 1,3 Dicarboxy-methyl-2-imino-imidazolidine 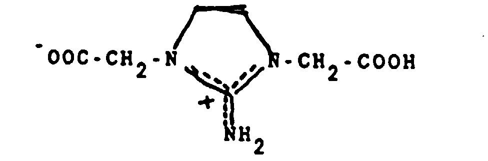
c) 2-Methyl-N,N'-dicarboxymethyl-imidazole 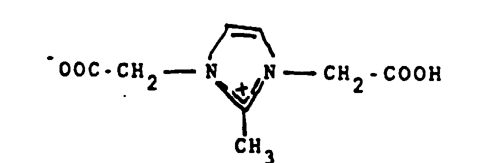
d) Epoxycreatine

3. Compounds generated by Walker and co-workers to inhibit creatine kinase, such as homocyclo-creatine, cyclocreatine
4. Small organic molecules which inhibit the purine metabolic enzyme, such as:
- cyclopropanone
- 2,3 butanedione
- cyclopropane
- salicylate derivative
- iodoacetamidosalicylate
- N-iodoacetamidoethylamino-naphthalene-1-sulphonate
- 1-sulphonate
- (4-iodoacetamidophenyl) amino-naphthalene-2-sulphonatea
- iodoacetanide derivatives
- butanedione
- 1 floro 2,4 dinitrobenzene
- 5,5', dithiobis (2-nitrobenzoic acid)
- benzene derivatives (e.g., fluorodinitrobenzene)
- N-cychohexyl-N'-beta (4-methyl-morpholine)

Derivatives or analogues of these molecules which will make them specific to CKB by linking them covalently to the substrates of CKB (i.e., creatine or ADP/ATP) or their metylene derivatives.
5. derivatives of ADP,ATP, such as:
a) 2,3-dialdehyde derivatives of ADP,ATP
b) ATP-γ(N-(2 chloroethyl)-N-methyl) amide (a mustard derivative)
c) imidazolides of AMP, ADP, ATP

6. A natural inhibitor in the serum of some patients, such as those with muscular dystrophy (which is a small dialyzable molecule of still unidentified origin).
7. Compounds which uncouple of creatine kinase from the complex: adenylate kinase/translocase/creatine kinase
8. Some of the blockers used in resolving a myocardial infarct are thought to inhibit creatine kinase.

### Inhibitors of Adeylate Kinase

1. Multi substrate inhibitors (Valentine, N., et al., Am. J. Hematol., 32(2) 143-145 (1989); Gustav, E., et al., JBC, 248:1121-1123 (1973)).
   a) P¹, P⁵-Di(adenosine-5') pentaphosphate
   b) the above compound in which methylene replaces some or all of the phosphate groups, facilitate uptake by cells.
   c) AP₄A P₁, P₄(diadenosine 5') tetraphosphate and its methylene substitutions
2. Elemental sulfur and its derivatives (Conner, J. and P.J. Russel, Research Communications, 113(1):348-352 (1983)).
3. Adriablastin
   (Toleikis, A.I, et al., Biokhimiia, 53(4):649-654 (1988)).
4. 5,5¹ dithiobis-2(nitrobenzoate)
   (Declerck, P.J. and M. Muller, Comp. Biochem. & Physio., 88(2):575-586 (1987)).
5. Adenosine, di-, tri-, tetraphosphopyridoxals
   (Yasami, T., et al., FEBS-LEH, 229(2):261-264 (1988)).
6. Potassium ferrate
   (Crivellone, M.D., et al., J. Biol. Chem., 260(5):2657.2661 (1985)).
7. 8-Azido-2'-O-dansyl-ATP
   Chuan, H., et al., J. Bio, Chem., 26(14):7981-7988 (1989)).

### Inhibitors of Adenosine Kinase

1. -5-iodotubercidin (from sponge of genus Echinodictyum)
   Weinberg, J.M., et al., Am. J. Physiol., 254(3p+2) pF311-322, (1988)).
2. From marine sources
   two very potent inhibitors
   4-Amino-5-bromo-pyrrolo
   [2,3-d] pyrimidine
5'-Deoxy-5-iodotubercidin
   (from red alga Hypnea Valentiae)
   (Davies, L.P., et al., Biochem. Pharm., 33(3):347-355 (1984)).
3. Clitocine and its derivatives
   (Moss, R.J., et al., J. Med. chem., 31(4):786-790 (1988)). Combinations of compounds inhibiting two of the above enzymes.

### Growth Factor Inhibition in Transformed or Virally Infected Epithelial Cells

For non-transformed mammalian cells to grow in vitro, they require the presence of appropriate polypeptide growth factor(s) that exert their effects through specific plasma membrane receptors. Primary epithelial cells are hard to grow in culture. This has hindered the study of epithelial cell transformation in vitro, and explains why most in vitro transformation experiments have been done with fibroblasts, although malignant tumors of non-epithelial cell origin account for only 10-20% of human neoplasms. However, human and rodent primary epithelial cells can be transformed with adenovirus, a DNA tumor virus (Houweling, A.P. et al., Virology 105:537-550 (1980); Vander Elsen, P.J. et al., Virology 131:242-246 (1983); Whittaker, J.L. et al., Mol. cell. Biol. 4:110.116 (1984)).

Adenoviruses normally infect quiescent epithelial cells and the expression of the adenovirus type 5, E1a 12S gene product enables primary rodent epithelial cells to proliferate in the presence or absence of serum (Quinlan, M.P. and T. Grodzicker, J. Virol. 61:673-682 (1987)). Thus, the 12S gene product provides a means of studying the changes involved in the immortalization and transformation of primary epithelial cells. The 12S gene is a member of the adenovirus E1a transcription unit. At early times, after infection, and in transformed cells, two E1a transcripts designated 13S', 12S' mRNAs are produced. These mRNAs are translated into proteins of 289 and 243 amino acids, respectively, that-differ only by the presence of an additional internal 46aa in the 289aa protein.

The products of the E1a region can immortalize primary cells (Moran, E. et al., J. Virol. 57:765-775 (1986)) and can cooperate with other viral genes and cellular oncogenes to transform primary rat cells (Ruley, H.E., Nature 304:602-606 (1983)). The E1a 12S protein seems to play a major role in the stimulation of cell proliferation responses. The 12S gene product is required for optimal virus production in growth arrested permissive cells, but not in actively growing cells (Spindler, K.R. et al., J. Virol. 53:742-750 (1985)). It induces cellular DNA synthesis and cell cycle progression in quiescant cells (Quinlan, M.P. and T. Grodzicker, J. Virol. 61:673-682 (1987); Spindler...ibid); Stabel, S.P. et al., EMBO J. 4:2329-2336 (1985)). It can immortalize primary epithelial cells so that they retain many of their differential characteristics.

The 12S product has been shown to cause/trigger the production of a growth factor, when introduced into primary kidney quiescent cells. Such a DNA tumor virus factor is a substance produced by or associated with a DNA tumor virus in a transformed or tumor cell and is capable of inducing immortalization in the absence of the DNA tumor virus itself. For example, recently Quinlan et al. (Quinlan, M.P. et al., PNAS 84:3283-3287 (1987)), found that infection of primary baby rat kidney cells with an adenovirus variant that encodes only the 12S product results in production of a growth factor that stimulates primary epithelial cells to proliferate. Media from cells that were infected with this variant virus, was filtered (to remove intact virus) and fed to cells which never saw the virus. Results showed that this conditioned media was able to induce epithelial cell DNA synthesis and proliferation between 24 and 36 hours after addition. A growth factor seems to be secreted from the epithelial cells upon infection with the 12S virus. The factor acts in an autocrine fashion and has a large molecular weight. The growth factor appears to be a unique mitogen for epithelial cells. Although it has been shown that the 12S product of adenovirus can induce DNA synthesis, proliferation, immortalization or transformation of its host cell, it is unclear how induction occurs. Other DNA tumor viruses associated with malignancies share sequence resemblances to the 12S protein of adenovirus and most probably secrete similar factors.

It appears that the growth factor released by 12S adenovirus infection might be mediating the seen observed effects of the 12S product (immortalization, induction of DNA synthesis). As described previously, the 12S product is capable of inducing the expression of CKB. The regions required to induce CKB expression or the production of the viral factor are similar. This suggests that the virus might be affecting the host cell genes via the secreted factor. Hence, the factor which is the hormone might be the real inducer of CKB and other enzymes. This is further favored by the fact that the kinetics of induction of CKB and the factor are similar. It is reasonable to expect that this factor (or derivatives thereof) is secreted by many tumors in which CKB (and adenosine metabolic enzymes) is highly activated. Further support for this idea is the fact that many DNA tumor viruses, such as polyoma and papilloma virus encode transforming products with amino acid sequences similar to the amino acid sequence of the adenovirus 12S products. Thus, cells infected with such viruses are very likely going to express this DNA tumor virus factor or transforming factor and cause induction of purine metabolic pathway exzymes in much the same manner as the 12S factor causes induction.

The CKB gene is known to be very responsive to multihormonal signals, implying it is indeed active in signalling or is along a metabolic path that mediates these effects. Thus, this adenovirus factor might be a new hormone which also regulates CKB. Most of these hormones, in spite of being different and having different receptors, might pass by or affect a common event which CKB is part of.

Even a seemingly unrelated virus, the cytomegalovirus, which induces CKB expression (Gonczol, E. and S. A. Plotkin, J. Gen. Virol., 65:1833.1834 (1984), also appears to induce production of a factor capable of stimulating DNA synthesis and modify cell growth. This factor appears to function, at least partially, by modifying microtubule structure. Creatine kinase is thought to associate with the microtubule structure. This factor might be the inducer of CKB expression.

Interruption of the autocrine loop which links this growth factor and its induction of enzymes, using selected drugs (e.g., competitive factor antagonists) can be carried out, using competitive antagonists which interfere with effects of the factor. In addition, antibodies against the factor or receptor it interacts with can be used to block the factor's effect. Such compounds can be used for the treatment of virally infected cells and tumors of epithelial origin.

### Application of the Present Invention

For the first time, induction of a cellular gene associated with energy metabolism by products of the transforming region of an oncogene has been demonstrated. In particular, induction of the brain creatine kinase gene by transforming domains of E1a has been demonstrated. As explained above, it has been shown that the product(s) of the transforming domain of an oncogene (e.g., E1a) induces expression from promoters of two cellular genes (brain creatine kinase, adenosine deaminase) encoding products which play critical roles in cellular energy metabolism and DNA synthesis. As also described, it is possible that these two enzymes are participants in a larger pathway in which other critical enzymes participate. It is possible that some of the enzymes have similar sequences, and are activated in a similar manner to that in which the two herein described genes are activated. If such oncogenes are transforming (effect transformation from a normal phenotype) because they modify or interfere with the pathway in which such enzymes participate, it is possible to design molecules (referred to as interfering substances) that will alter their activity. These molecules might counteract the viral or oncogenic activity and thereby reverse a transformed phenotype when administered into malignant cells. Among potential interfering compounds would be synthetic analogues that would inhibit these enzymes. For example, there exist transition-state substrate analogues of creatine phosphate (e.g., epoxycreatine, cyclocreatine, etc.) that will inhibit creatine kinase. Such compounds can be introduced into transformed cells, such as small cell lung carcinoma, retinoblastoma, pulmonary carcinoid tumors and others, and their ability to revert the transformed phenotype to a normal phenotype or their effect on the growth rate of the tumor cells will be assessed. It is also possible to make anti-gene constructs (e.g., in a retrovirus) for the brain creatine kinase gene and introduce them into cells, using known techniques, to suppress expression of brain creatine kinase. Local administration of such a compound in tumors might suppress the growth of malignant cells. It is also possible to modify adenosine uptake in malignant cells. The uptake of adenosine could be modified by these viral or oncogenic signals. The adenosine receptors and the ADA binding protein might be involved in receiving such signals. Interfering with adenosine uptake might modify the transmission of the signals. It is also possible to interfere with the 19 amino acid peptide whose sequence is common to many tumor viruses (described above). If this peptide is important for induction, directly or indirectly, then the administration of antibodies directed against this peptide or antagonist peptides could be used to neutralize the peptide's effects. The same could be done for NH₂ terminal region which is involved in DNA synthesis induction and CKB regulation.

Viral transformation is known to be the result of collaboration between two or more genes. For example, E1a products can immortalize primary cells, but in collaboration with an activated ras gene, they can transform cells fully. The activity of the ras gene product, p21, is tightly regulated by GTP/GDP pools. Preliminary experiments have shown that creatine kinase can contribute not only to the ATP/ADP pool maintenance, but might also extend its activity to catalyze the transfer of high energy phosphate from creatine phosphate to DGP to GTP. The G-binding protein family, including p21, is involved in signal transduction and is regulated by GTP/GDP ratios. If brain creatine kinase has a role in regulating GDP/GTP pools, directly or indirectly, then brain creatine kinase will be a regulator of these signal transduction pathways. Thus, interfering with brain creatine kinase, as described above, would also result in interference with these pathways. The induction of CKB expression by E1a in the nucleus might result in activation of nucleotide pools needed to charge or activate some nucleotide binding proteins (e.g., ras oncogene product). The ras oncogene, once activated by binding GTP, will continue the proliferative signalling pathway of transformation. This hypothesis might explain a collaboration between nuclear and cytoplasmic oncogenes. Where nuclear oncogenes turn on expression of products needed for cytoplasmic events. Here, too, regulation of brain creatine kinase activity in transformed cells, and, hence, of nucleotide pools, would result in suppression of the rapid proliferative signal and present a transformed phenotype.

It is now possible to intefere with, inhibit or reduce expression from the promoter of a cellular gene (such as a gene encoding an enzyme which participates in adenosine metabolism) which is induced or activated by a viral product. In particular, it is now possible to interfere with the activity of the oncogenic products of the adenovirus E1a region. That is, it is possible to block induction of both creatine kinase levels and mRNA levels by the oncogenic products of domains 1 and 2 of the E1a region by several routes. For example, expression of the domain can be inhibited using known anti-sense oligonucleotide techniques. For example, an oligonucleotide sequence complementary to all or a portion of domain 2 can be introduced into cells; hybridization of the introduced oligonucleotide results in inhibition of activity of domain 2. As used herein, a nucleotide sequence which is sufficiently similar to the sequence of the CKB promoter region that a viral product interacts with it as the viral product interacts with CKB, is a functional equivalent, and similar inhibition strategies may be employed.

It is also possible to interfere with or inhibit the activity of the enzymes of this pathway. For example, substrate analogues (e.g. epoxycreatine, homocyclocreatine) can be administered to inhibit enzymes involved in regulation of nucleotide levels in the cell (e.g. CKB, ADA) by, for example, competing with creatine or creatine phosphate (native substrates) for the active site of creatine kinase. Transition state substrate analogues can be designed to inhibit the enzyme. Epoxy creatine, for example, inhibits the activity of CKB, and homocyclocreatine reduces the efficiency of the enzyme dramatically. A series of related compounds can be designed to represent variations of the substrates for this pathway, and their inhibitory effects tested. These suggested compounds are specific for one enzyme and should be interfere with other cellular processes.

The rapid proliferation of many cancer cells requires a higher metabolic rate, which in turn requires rapid regeneration of ATP for energy. Thus, it is possible that tumor-inducing viruses, such as adenoviruses, encode proteins, such as the E1a products, which can induce expression of creatine kinase or other enzymes involved in adenosine metabolism, to maintain high levels of ATP in infected cells. cAMP is a product of ATP and it too might be activated. The present invention makes it possible to counter the effects of the adenovirus oncogenic products, and other related viral products provides a method for regulating creatine kinase expression or activity, and thereby makes it possible to control the rate of proliferation of cells containing adenovirus or another tumor virus. As mentioned, the creatine kinase promoter region and the adenosine deaminase promoter show striking sequence similarity (See Figure 9). Both creatine kinase and adenosine deaminase participate in adenosine metabolism and are tumor markers. It is possible that expression from the adenosine deaminase promter is controlled in a similar manner by adenovirus oncogenic products. It is also possible that adenosine deaminase induction can be controlled in a similar manner. Other transforming viruses, (e.g., SV40 larg T, papilloma virus), which are known to share sequence similarity with domain 2 may also have the ability to affect induction of creatine kinase or another cellular enzyme involved in energy metabolism. Induction by such transforming viruses might also be important for regulation of intermediary metabolic events which occur after oncogenic activation. For example, early viral products of both RNA and DNA tumor viruses might modify uptake of adenosine by receptors.

Because of the increased levels of CKB and other cooperative enzymes, simple enzyme assays can be used to detect a DNA tumor virus in cells in which one is thought to be present. This can be used for diagnostic purposes or for monitoring treatment provided to an individual in which the presence of transformed cells has been detected. For example, a diagnostic assay can be carried out as follows: proteins can be extracted from cervical scrappings, such as those obtained for cytologic evaluation and extracted (e.g., by freeze-thawing). The supernatant can be assayed for enzymes of interest (e.g., creatine kinase, adenylate kinase). Two or more enzymes can be assessed simultaneously. Presently-available methods, such as Southern blots which use labelled probes from the virus, are time consuming and costly. Enzyme assays, however, are simple and can be carried out quickly (e.g., in this case, approximately 25 minutes). Such enzyme assays can measure, for example, CKB activity, as well as activity of one or two additional enzymes along the purine metabolic pathway.

It may be that the sensitivity of cytologic examination (Papanicolaou smear test) carried out in conjunction with detection of human papillomavirus is superior to the use of either cytologic studies or human papillomavirus detection alone in evaluating patients with cervical lesions (Ritter, D.B. et al., Am. J. Obst. Cynecol., 159(6):1517.1525 (1988)). Through the method of the present invention, detection of infection with HPV will be a routine assay which can compliment Papanicolaou smear test. Enzyme assays can be substituted for Southern blotting, in order to detect viral activity. For example, a simple complementation test which is practical, inexpensive and easy to carry out in clinical settings, can be used.

In the method of the present invention, a drug or compound which: 1) is able to enter cells in which a DNA tumor virus or a DNA tumor virus factor has acted upon cellular genes encoding purine metabolic enzymes or their products and, as a result, increased expression of that gene and 2) 'is able to prevent the transforming domain of the oncogenic virus from inducing cellular genes or to inhibit the increased activity of induced cellular genes, is administered to an individual in whom a DNA tumor virus or virus factor has acted, resulting in a cellular abnormality or a tumor. The drug or compound is administered in sufficient quantity to reduce or eliminate the effect of the DNA tumor virus or virus factor on cells. Drugs useful in the present method can be selected or designed to act in one or more of the above-described ways. For example, one useful type of drug is one which inhibits the purine metabolic enzyme(s) whose activity in transformed cells is increased. Inhibition will result in a decrease in the activity of the enzyme(s) and/or will interfere with the ability of the purine metabolic enzymes to associate or interact with one another. Alternatively, a drug which is an antisense construct (oligonucleotide sequence) which binds selectively to a region of the RHA necessary for translation can be used. In the case of CKB, the oligonucleotide sequence is one which binds selectively to a region of CKB RNA, as represented in Figure 12, which is needed for translation. The effects of the transforming viral DNA are, as a result, reduced or prevented. That is, an oligonucleotide sequence capable of entering the cell nucleus and binding (hybridizing) to the specific activated message to block it can be introduced into cells. Hybridization to the nucleotide sequence renders it unavailable for further activity. In another approach, a drug which interferes with a transcription factor (e.g., by acting upon a promoter or blocking the ability of a transcription factor(s) to activate a promoter(s) to transcribe) is administered in sufficient quantities to have the desired effect. Alternatively, a drug which inhibits interaction of the viral oncogenic product(s) (e.g., the E7 product, E1a product) with a cellular gene encoding a purine metabolic enzyme can be administered. Inactivation can occur, for example, by binding of the drug to the viral oncogenic product or by destruction of the viral oncogenic product by the drug. A variety of presently-available compounds (described below) can be assessed for their ability to inhibit creatine kinase or adenylate kinase, which seems to work in conjunction with creatine kinase and can serve as models for design of additional drugs for creatine kinase inhibition.

In the method of the present invention, at least one drug which is capable of inhibiting adenylate kinase, another enzyme which participates in the purine metabolic pathway or a cellular component which cooperates with a purine metabolic pathway enzyme is administered to an individual in a manner appropriate for introducing the drug into affected cells. The form in which the drug(s) will be administered is determined by the type of drug used, as will the route of administration and the quantity given.

For example, in the case of an individual in whom cervical dysplasia or cervical carcinoma is present, a compound or drug which is able to inhibit the activity of the HPV oncogenic products and, thus, inhibit modulation of the cellular gene (e.g., the CK gene, adenylate kinase gene) is administered in a therapeutically effective quantity. Such a compound or drug can be, for example, a peptide which binds domains 1 and 2 of E1a or E7, to prevent them from inducing their transactivation activity. Alternatively, they can be inhibitors of the activated cellular enzymes. In this instance, as well as in other instances of epithelial tumors, the compound or drug can be administered topically or can be injected, infused or otherwise introduced.

Compounds or drugs used for this purpose can be existing substances, analogues of existing substances or substances designed specifically to interfere with the action of the oncogenic product. The following is a description of existing compounds known to inhibit creatine kinase and existing compounds known to inhibit adenylate kinase. It will be possible to modify the below described compounds to produce analogues which have enhanced characteristics, such as greater specificity for the enzyme or the oncogenic product, enhanced stability, enhanced uptake into cells, tighter binding to the enzyme or the oncogenic product or better inhibitory activity. In addition, based on knowledge of the structural and functional characteristics of the oncogenes and of the cellular genes upon which the oncogenic products act, it is possible to design other compounds or drugs useful in the present method.

A further application of the work described herein is identification of existing compounds inhibiting and counterbalancing the induction effects caused by these DNA tumor viruses in the cervix. It is clear that CKB is an enzyme that is very active in many tumors, particularly those of epithelial origin (e.g., small cell carcino-ma, retinoblastomas, cervical carcinomas, bladder cancer, ovarian cancer and many more). In the cervix, the presence of persistent disease has been shown to correlate with a raised CKB level (Rubery, E.D. et al., Eur. J. Cancer Clin., 18:951-956 (1982); Silverman, L.M. et al.). CKB is found in the cytoplasm of both benign and malignant glandular epithelium. In malignant tissues, the level increases and CKB is secreted or released. (Z.A., Clin. Chem., 25:1432.1435 (1979)).

Adenylate kinase is another purine metabolic enzyme which can be inhibited by the present method and whose inhibition will result in inhibition of cellular transformation. Adenylate kinases (NTP: AMP phosphotransferases, N, adenine or guanine) are relatively small, monomeric intracellular enzymes (MWt 21-27 KDa) which catalyze the interconversion of nucleotides according to the equation: Hg⁺⁺ NTP + AMP Hg⁺⁺ NDP + ADP. The enzyme is ubiquitous and particularly plentiful in tissues where the turnover of energy from adenine nucleotides is high (Noda, L., In: Enzymes, (Boyer, P., ed.) Vol. 8, pp. 279-305, Academic Press, Orlando, F1). It has an important role in maintaining energy charge of the adenylate pool (Lipman, F., Curr. Top Cell. Regul., 18:301-311 (1981); Atkinson, D.E., Biochemistry, 7:4030-4034 (1968)). The prokaryotic gene from E. coli has been cloned (Brune, M. et al., Nucleic Acids Res., 13:7139-7151 (1985). This and previous studies (Glaser, M. et al., J. Bacteriol., 123:128-136 (1975) have shown that adenylate kinase is a key enzyme in controlling the rate of cell growth. Konrad et al. (Konrad et al., J. Biol. Chem., 263:19468-19474 (1988)) have cloned the eukaryotic gene from budding yeast. By gene disruption experiments they show that the cytosolic gene is needed for normal cell growth but is not essential for cell viability.

The fact that creatine kinase associates with the adenylate kinase to form a comparticle to regulate ATP levels, makes it important to try to intervene with both. Using a combination of drugs that inhibit both enzymes might be an excellent way to reduce ATP levels and growth of tumors. These conbinations might generate a new family of antiviral and antitumor drugs.

The invention will now be illustrated by the following Examples, which are not to be taken as limiting in any way.

### EXAMPLES

### Example 1 Isolation of a Clone for Human Brain Creatine Kinase Gene

DNA Methods. The 180-bp rabbit cDNA probe (M180) used for screening was prepared by primer extension (with Klenow fragment, Boehringer Mannheim) of an M13 mp8 clone using [³²P]dATP (Amersham Corp.) as the radioactive nucleotide and purified. Maniatis, T. et al., Molecular Cloning, A Laboratory Manual, Cold Spring Harbor Laboratory, Cold Spring Harbor, NY (1982). This probe covers codons 256-316 which encompass a stretch of 12 amino acids (including the reactive Cys-283) that are well conserved between the creatine kinase isozymes. Watts, D.C., The Enzymes, VIII:383-455 (1973); Kumar, S.A. and A.J.L. Macario, Hybridoma, 4:297-309 (1985).

A human lymphocyte-derived genomic library in the Charon 4A lambda phage vector (kindly provided by Dr. D. Page, Department of Biology and Whitehead Institute, MIT) was screened with the 180-bp probe. Approximately 10⁶ plaques were screened by the hybridization method. Maniatis, T. et al., Molecular Cloning, A Laboratory Manual, Cold Spring Harbor Laboratory, Cold Spring Harbor, NY (1982). Twenty-four potential positive clones were isolated, and clone 16B2 was chosen for further studies. Clone 16B2 gave the strongest hybridization signal and contained a 12-kbp insert of human DNA. This insert was cloned into plasmid pBR322 to give plasmid pHCK302 (Fig. 1). The M180 probe hybridized to a 0.9-kbp PstI fragment (Fig. 1) and shares with it 95% sequence identity. Analysis of the sequence of this subclone revealed a replacement of Val-280 to isoleucine, a substitution known to distinguish the B from the M isozyme in different species. Watts, D.C., The Enzymes, VIII:383-455 (1973).

End-labeled oligonucleotides for S1 mapping were extended with Klenow enzyme and the four deoxynucleotide triphosphates, using M13 clones as templates; the products were digested with BamHI and purified as discussed elsewhere. Kingston, R.E. et al., Mol. Cell. Biol., 6:3180-3190 (1986).

DNA sequencing was performed by the dideoxy chain termination method, using the M13 universal primer as well as a variety of synthetic oligonucleotides. Klenow fragment and avian myeloblastosis virus reverse transcriptase were used interchangeably with ³²P or ³⁵S as the label. Reactions were run at 37.50°C, depending on the GC content of the sequence. In one case, the chemical method was also used. Sanger, F. et al., Proc. Natl. Acad. Sci., USA, 74:5463-5467 (1977); Sanger. F. et al., J. Mol. Biol., 143:161-178 (1980); Maxam, A.M. and W. Gilbert, Methods Enzymol., 65:499-559 (1980).

### Cell Culture, Transfection, and Expression of Creatine Kinase Activity.

Mouse BALB/c/3T3 fibroblasts, and LtK and human HeLa cells were cultured in Dulbecco's modified Eagle's medium supplemented with 10% calf serum in 10-cm plates. Transfections were carried out using a modification of the calcium phosphate precipitation procedure. Kingston, R.E. et al., Mol. Cell. Biol., 6:3180-3190 (1986); Graham, F.L. and A.J. van der Eb, Virology, 52:456-467 (1973). Ten µg of DNA were used per transfection; this consisted of 2-8 ug of plasmid pHCK302 or pHCK201, 1 ug of plasmid pXGH5 (a reporter plasmid), and the remainder as plasmid pUC13. Plasmid pXGH5 is a chimeric gene in which the human growth hormone coding region is fused to the mouse -MT-I promoter. Selden, F.R. et al., Mol. Cell. Biol., 6:3173.3179 (1986); Hamer, D.H. and M. Walling, J. Mol. Appl. Genet., 1:273-288 (1982); Denoto, F.M. et al., Nucleic Acids Res., 9:3719-3730 (1981); Durnam, D.M. et al., Proc. Natl. Acad. Sci., USA, 77:6511-6515 (1980). The human growth hormone gene product is secreted, and the amount produced can be quantitated by a radioimmune sandwich assay to measure transfection efficiency. Selden, F.R. et al., Mol. Cell. Biol., 6:3173-3179 (1986).

Cells were harvested 48 hours after transfection for either protein assays or RNA analysis. For protein work, the cells were thawed by cycles of freeze-thawing. The cell-free supernatant was subsequently assayed for creatine kinase activity using a coupled reaction in which rates are reflected by the accumulation of NADPH as monitored at 340 nm. Eppenberger, H.M. et al., J. Biol. Chem., 242:204-209 (1967). In addition, approximately 20 ug of total protein from each extract was analyzed by immune blots with a mouse anti-human B isozyme monoclonal antibody (Hybritech). Burnette, W.N., Anal. Biochem., 112:195.203 (1981). RNA Preparation and Analysis. Total cellular RNA from transfected cells was prepared by the guanidinum/CsCl method. Maniatis, T. et al., Molecular Cloning, A Laboratory Manual, Cold Spring Harbor Laboratory, Cold Spring Harbor, NY (1982); Ullrich, A. et al., Science, 196:1313.1318 (1977). S1 nuclease analysis was performed as described. Berk, A.J. and P.A. Sharp, Cell, 12:721-731 (1977). For primer extension analysis, the ³²P-end-labeled oligonucleotides were hybridized overnight at 30°C to 30 ug of total RNA, and the hybrids were precipitated and washed. Extension with avian myeloblastosis virus reverse transcriptase in the presence of unlabeled nucleotides was carried out at 42°C for 90 minutes. This was followed by ribonuclease A digestion, two phenol/chloroform extractions, and precipitation. Products were analyzed on 6% acrylamide gels.

### Results

Rabbit B isozyme cDNA probes were used to determine whether clone 16B2 encodes the remainder of the human enzyme. Pickering, L. et al., Proc. Natl. Acad. Sci., USA, 82:2310-2314 (1985). Probe H12-8 from the amino-terminal and probe B-19 from the carboxyl-terminal coding region hybridize to the human sequence (Fig. 1). They are separated by 2.1 kbp, which is considerably greater than the distance (443 bp) between the closest end points in the rabbit B isozyme cDNA. Because the human and rabbit enzymes have similar molecular weights, we assume that the enlarged spacing in the human gene is due to intervening sequences. Watts, D.C., The Enzymes, VIII:383-455 (1973).

Sequence analysis of the region that hybridizes to probe H12-8 reveals an ATG and additional sequences which encode a protein that is identical to the amino terminus of the recently published human B isozyme cDNA. Kaye, F.J. et al., J. Clin. Invest., 79:1412-1420 (1987). Similarly, the sequence of the portion that hybridizes to B-19 (Fig. 1) shows that this region encodes a protein that is identical to the carboxyl terminus of the human cDNA and that the TGA stop codon is located in the expected position. Kaye, F.J. et al., J. Clin. Invest., 79:1412-1420 (1987). There also is exact identity over the entire 184 nucleotides of 3'-flanking sequence in the cDNA with the sequence encoded by the genomic DNA. These data show that the entire coding sequence for human brain creatine kinase is encompassed by a region of 2.5 kbp that is embedded within the 12-kbp genomic fragment that was isolated from the initial screen of the library.

### Transient Expression of the Human Brain Creatine Kinase Clone.

Plasmid pHCK302 which contains the 12-kbp genomic fragment (Figure 1) expressed a functional brain creatine kinase when transfected into BALB/c/3T3, LtK⁻, and human HeLa cells. The subclone pHCK 201 (Figure 1), which has only 850 bp of 5'-flanking sequences, also expressed the enzyme to comparable levels. The amount of enzyme activity produced is approximately in proportion to the amount of plasmid transfected, up to 10 ug of pHCK 201/10-cm plate transfection. (Samples were normalized for protein content in each assay). A mouse anti-human B isozyme monoclonal antibody can detect the human enzyme in extracts of transfected cells. It was not possible to detect any level of the enzyme in untransfected LtK cells. It has been observed that, in transient expression experiments, genes which are silent in the host can be expressed if introduced exogenously. Melton, D.W. et al., Proc. Natl. Acad. Sci. USA, 81:2147-2864 (1984). Similar results were obtained with transfection into HeLa and BALB/c/3T3 cells.

### Analysis of the 5'-Flanking-Region.

The published cDNA sequence of the human B isozyme (isolated by using our probes) extends to only 8 nucleotides on the 5'-side of the ATG initiator codon. Kaye, F.J, et al., J. Clin. Invest., 79:1412-1420 (1987). To identify the promoter element of the gene for the B-isozyme, a variety of synthetic oligonucleotides were constructed and used to sequence the 5'-flanking region and to map the transcriptional start site (Figure 2A). The promoter element appears to be within the first 850 base pairs of 5'-flanking sequences because plasmid pHCK201 expresses functional enzyme. As shown below, a comparison of primer extension with S1 nuclease mapping experiments reveals the presence of a 230-bp intron that is 12 bp upstream of the ATG initiation codon. A 5'-end of B isozyme mRNA that, in the gene, is 310 base pairs from this ATG codon was identified.

Figure 2B shows results of primer extensions of an oligonucleotide (oligomer 1 (Figure 2A)) that is complementary to a 24-nucleotide segment which starts 21 nucleotides after the A of the ATG initiator. Hybridization (to mRNA) and extension of this primer gave two fragments of 128 and 132 nucleotides that appeared with the nonexpressing LtK⁻ cells that were transfected with plasmid pUC13 (lane 1). An intense doublet corresponding to fragments of 126 and 128 nucleotides appeared when RNA was used from LtK⁻ cells transfected with pHCK201 (lane 2). (A similar result was obtained when pHCK201 was substituted with pHCK302 in the transfections (data not shown)). RNA from the human monocyte cell cline U937, which endogenously expresses the B isozyme, gave the same bands as were seen with LtK⁻ cells transfected with plasmid pHCK201 (lane 3). This implies that the isolated gene is expressed from the same promoter as is the endogenous gene. The size of the primer extension product indicates that the mRNA has about 81 nucleotides of 5'-untranslated sequence.

A 5'-end-labeled single-stranded DNA probe (Probe I (Figure 2A)) was used for S1 nuclease mapping. RNA from the monocytic U937 cell line and from LtK⁻ cells transfected with either plasmid pHCK201 or plasmid pHCK302 gave a major protected fragment estimated as 192 nucleotides (Figure 2C, lanes 4, 6, 7 and 8). This band was absent in LtK⁻ cells transfected with plasmid pUC13 (lane 5). After subtracting the 180 nucleotides of coding region that is encompassed by the probe, the data imply that there are 12 additional nucleotides that extend to either the transcript start site or to an intron-exon junction. In view of the results of the primer extensions, an intron evidently starts 12 bp before the ATG initiator codon. This also suggests that, if there is just one intron in the 5'-flanking region, the the first exon is estimated as 69 (81 - 12) bp in length.

A single-stranded end-labeled probe (Probe II (Figure 2A)) was used in S1 nuclease analysis (with mRNA from LtK⁻-transfected cells) to determine whether an mRNA is encoded by the region that is upstream of the intron. Comparison of the resultant protected fragment with a "sequencing ladder" of Probe II (data not shown) revealed the start site(s) which is marked in Figure 3A. This is located at 67 nucleotides before the splice donor site.

To confirm these results, a 60-mer oligonucleotide (oligomer 3) was synthesized that encompass the putative start site. This oligonucleotide was end-labeled and used for S1 nuclease mapping, using RNA from pHCK201-transfected LtK cells. Figure 2D (left lane) shows the protected band that is displayed next to a "G ladder" obtained by Maxam-Gilbert sequencing of the labeled oligonucleotide. Maxam, A.M. and W. Gilbert, Methods Enzymol., 65:499-559 (1980). The major 5'-end maps to the C that is 69 bp before the intron. It was concluded that the 5'-end starts at sites that are 67-69 bp upstream of the introns. These sites are underlined in Figure 3A, in which the central G is arbitrarily numbered +1.

### Example 2 Determination of Effect of Adenovirus E1a Domain 3 on Brain Creatine Kinase Expression

Results of the following work demonstrated that a point mutation in domain 3 that is defective for induction of E₂E can induce expression of brain creatine kinase.

HeLa cells were grown in dishes in Dulbecco's Modified Eagles media (DMEM) supplemented with 10% FCS (fetal calf serum). Close to confluency, the cells were washed with serum-free media and infected for one hour with 20 pfu/cell with pm975 wild-type Ad5 virus (encodes the 13S product) or with the pm975-1098 mutant which has a point mutation in domains 3. J.W. Lillie et al., Cell, 46:1043 (1986); J.W. Lillie et al., Cell, 50:1091 (1987). AraC was added at a concentration of 20 µg/ml post-infection and was replenished every 8-10 hours. Hock-infected plates (control plates) had similar treatment but lacked infection with virus. At 4, 7, 24, and 30 hours post-infection, total cellular RNA was harvested, purified and 30 µgs were hybridized to an end-labeled probe that is specific for CKB (oligomer I, panel A, Figure 4) or for E₂E (18 nt, panel B, Figure 4). The positions of these probes relative to the two genes are indicated below each autoradiogram shown in Figure 4, and they give primer-extended bands of 125 nucleotides (A) and 68 nucleotides (B), respectively. Primer extension was carried out on 30 µg of total cellular RNA using liquid hybridization conditions at 30°C. J.W. Lillie et al. Cell, 46:1043 (1986). Panel A shows induction of CKB mRNA (lanes a-d are for the wild-type virus and lanes e-h are for the mutant). Lane i depicts the endogenous level of CKB in the HeLa cell line that was used (24 hours mock infected plate) and the level does not change with time. Panel B shows the induction of E2E RNA (lanes j-m are for wild-type virus and lanes n-q are for mutant). Lane r is an analysis of the RNA isolated from the mock-infection and shows no endogenous E2E RNA. Panel C shows the results of measurements of creatine kinase activity when cells were infected with wild-type and mutant virus at 5 pfu/cell and 50 pfu/cell. The cells were harvested for protein at 16, 24, 36, and 40 hours post-infection by cycles of freeze-thawing and assayed for creatine kinase activity using a kit from Sigma Chemical Company (St. Louis, MO). Activity is reported as a change in absorbance at 340 nm per min per mg protein. O-CKB activity in mock infected plates,●- in wild-type virus infected plates, and ▲- In plates infected with pm975-1098 mutant.

### Example 3 Determination of Effect of the 12S Product of E1a Which Lacks Domain 3 on Expression of Brain Creatine Kinase

Results of the following work demonstrated that the 12S product of E1a which lacks domain 3 can activate expression of brain creatine kinase.

HeLa cells were infected, as described in Example 2 at 20 pfu/cell with virus expressing the 12S. (d11500) or 13S (pm975) products. J.W. Lillie et al., Cell, 46:1043 (1986); J.W. Lillie et al., Cell, 50:1091 (1987). Total cellular RNA was harvested at 36 and 42 hours post-infection and 30 µg were probed for brain creatine kinase mRNA by using the primer extension probe shown in Figure 4. Three results are shown in Figure 5: Lanes a and b show results for mock infection, lanes c and d are for RNA that was harvested from dl1500-infected plates and lanes e and f show results of probing RNA that was harvested from cells infected with the pm975 virus.

### Example 4 Determination of Effect of Adenovirus E1a Domain 2 on Brain Creatine Kinase Expression

Results of the following work demonstrated that point mutations in domain 2 cause impairment of E1a-induced expression of brain creatine kinase.

HeLa cells were infected with wild-type or mutant viruses as described in Example 2, and RNA was harvested at 20 and 40 hours post-infection and probed for brain creatine kinase (Figure 6, panel A) or E₂E (Figure 6, panel B) by use of primer extension probes (See Example 2). Infections were done with the following viruses shown in Figure 6: lane a, mock infection; lanes b, g, wild-type pm975 virus; lane c, mutant d1312 virus (deleted for the E1a gene); lanes d, e, h, i, mutant viruses pm975-936 and 953 each of which has a point mutation in domain 2; lanes f, j, mutant virus pm975-1098 which has a point mutation in domain 3.

### Example 5 Determination of Effect of Adenovirus E1a Domain 1 on Brain Creatine Kinase Expression

Amino terminal deletions into domain one obliterate the ability of E1a to induce CKB expression. HeLa cells were infected as described in Example 2 with wild-type pm975 virus or the amino terminal deletions generated in Harlos's lab. The names of the deletions are indicated with reference to the exact amino acids removed. The bars represent creatine kinase activity in infected cells. As deletions get into domain one creatine kinase activity induction is lost.

## Claims

1. A method of inhibiting transformation of a mammalian cell in which: (A) activity of a purine metabolic enzyme is increased, comprising inhibiting activity of the purine metabolic enzyme, the purine metabolic enzyme for example being selected from the group consisting of: creatine kinase, adenylate kinase, adenylate cyclase and adenosine kinase; or (B) creatine kinase activity is increased, comprising introducing into the mammalian cell a compound which reduces activity of creatine kinase, optionally further comprising inhibiting the activity of at least one purine metabolic enzyme which acts in conjunctions with creatine kinase wherein for example the purine metabolic enzyme which acts in conjunction with creatine kinase is selected from the group consisting of: adenosine kinase; adenylate kinase and adenylate cyclase.

2. A method of inhibiting transformation of a mammalian cell by a DNA tumor virus which increases activity of a purine metabolic enzyme in the mammalian cell or by a DNA tumor virus factor which increases activity of a purine metabolic enzyme in the mammalian cell, comprising a method selected from the group consisting of:
a) inhibiting activity of the purine metabolic enzyme in the mammalian cell;
b) blocking translation of RNA encoding the purine metabolic enzyme produced by the DNA tumor virus;
c) blocking a transcription factor, produced by the DNA tumor virus, which induces expression of the purine metabolic enzyme;
d) blocking a cellular transcription factor, activated by the DNA tumor virus, which induces expression of the purine metabolic enzyme;
e) inhibiting interaction of a product of the DNA tumor virus with a mammalian cell gene which encodes the purine metabolic enzyme;
f) inhibiting interaction of the DNA tumor virus factor with a mammalian cell gene which encodes the purine metabolic enzyme; and
g) inhibiting interaction of the DNA tumor virus with a cellular protein, the interaction resulting in regulation of activity of the purine metabolic enzyme in the mammalian cell, wherein for example: (a) the oncogenic product of a human papillomavirus is the product encoded by the E7 gene of a human papillomavirus selected from the group consisting of human papillomavirus 16, human papillomavirus 18, human papillomavirus 31, and human papillomavirus 33; and/or (b) the purine metabolic enzyme is selected from the group consisting of: creatine kinase, adenylate kinase, adenylate cyclase and adenosine kinase; and/or (c) blocking translation of RNA encoding creatine kinase is carried out by introducing into the mammalian cell an antisense construct which is an oligonucleotide sequence selected to bind to a region of the RNA encoding creatine kinase necessary for translation of the RNA.

3. A method of inhibiting transformation of a mammalian cell by a DNA tumor virus which increases activity of a purine metabolic enzyme in the mammalian cell, comprising introducing into the mammalian cell a compound selected from the group consisting of:
a) compounds which inhibit activity of the purine metabolic enzyme whose activity in the cell is increased;
b) compounds which bind selectively to a region of RNA encoding the purine metabolic enzyme which is induced by the DNA tumor virus, the region of RNA being necessary for translation of the RNA, thereby blocking translation of the RNA;
c) compounds which block a viral transcription factor which induces expression of the purine metabolic enzyme;
d) compounds which inhibit interaction of a product of the DNA tumor virus with a gene encoding the purine metabolic enzyme; and
e) inhibiting interaction of the DNA tumor virus with a cellular protein, the interaction resulting in regulation of activity of purine metabolic enzyme in the mammalian cell, wherein for example the purine metabolic enzyme is selected from the group consisting of: creatine kinase, adenylate kinase, adenylate cyclase and adenosine kinase.

4. A method of inhibiting transformation of a mammalian cell by a DNA tumor virus which increases activity of creatine kinase in the mammalian cell or by a DNA tumor virus factor which increases activity of creatine kinase in the mammalian cell, comprising a method selected from the group consisting of:
a) inhibiting activity of creatine kinase in the mammalian cell;
b) blocking translation of RNA encoding creatine kinase produced by the DNA tumor virus;
c) blocking a transcription factor, produced by the DNA tumor virus, which induces expression of creatine kinase in the mammalian cell; and
d) blocking a transcription factor, activated by the DNA tumor virus, which induces expression of creatine kinase;
e) inhibiting interaction of a product of the DNA tumor virus with a mammalian cell gene which encodes creatine kinase; and
f) inhibiting interaction of the DNA tumor virus factor with a mammalian cell gene which encodes creatine kinase, wherein for example (a) blocking translation of RNA encoding creatine kinase is carried out by introducing into the mammalian cell an antisense construct which is an oligonucleotide sequence selected to bind to a region of the RNA encoding creatine kinase necessary for translation of the RNA; and/or (b) the DNA virus is selected from the group consisting of human papovaviruses, adenoviruses and herpes virus; and/or (c) the human papovavirus is human papillomavirus.

5. A method of inhibiting in a cell the ability of an oncogenic product of a DNA virus to modulate expression of a selected cellular gene, comprising interfering with interaction of the oncogenic product with the promoter of the selected cellular gene, or a method of inhibiting, in a mammalian cell, modulation of a selected cellular gene which encodes an enzyme which participates in purine metabolism by an oncogenic product of a DNA virus, comprising introducing into the cell a drug which interferes with interaction of the oncogenic product with the promoter of the selected cellular gene, or a method of inhibiting, in an epithelial cell, modulation of expression of the gene encoding creatine kinase by an oncogenic product of a human papillomavirus, comprising introducing into the cells a drug which interferes with interaction of the oncogenic product with the promoter of the gene encoding creatine kinases, wherein for example the oncogenic product of a human papillomavirus is the product encoded by the E7 gene of a human papillomavirus selected from the group consisting of human papillomavirus 16, human papillomavirus 18, human papillomavirus 31, and human papillomavirus 33.

6. A method of detecting transformation of a mammalian cell by a DNA tumor virus or by a DNA tumor factor comprising determining the level of activity in the mammalian cell of at least one purine metabolic enzyme, wherein for example (a) at least one purine metabolic enzyme is selected from the group consisting of: creatine kinase; adenosine kinase; adenylate kinase and adenylate cyclase; and/or (b) the purine metabolic enzyme is selected from the group consisting of: creatine kinase, adenylate kinase, adenylate cyclase and adenosine kinases, and/or (c) the DNA virus is selected from the group consisting of: human papovaviruses, adenoviruses and herpes virus (e.g. human cytomegalovirus).

7. A method of detecting the presence of a DNA tumor virus in a mammalian cell comprising determining the level of activity in the mammalian cell of a purine metabolic enzyme, wherein an increased level of activity of the purine metabolic enzyme is associated with the presence of the DNA tumor virus, wherein for example (a) the purine metabolic enzyme is selected from the group consisting of: creatine kinase, adenylate kinase, adenylate cyclase and adenosine kinase, and/or (b) the DNA virus is selected from the group consisting of: human papovaviruses, adenoviruses and herpes virus (e.g. human cytomegalovirus).

8. A method of inhibiting metastasis of a mammalian cell in which activity of a purine metabolic enzyme is increased, comprising inhibiting activity of the purine metabolic enzyme, wherein for example the activity of the purine metabolic enzyme is inhibited by a method selected from the group consisting of:
a) inhibiting activity of the purine metabolic enzyme in the mammalian cell;
b) blocking translation of RNA encoding the purine metabolic enzyme produced by the DNA tumor virus;
c) blocking a transcription factor, produced by the DNA tumor virus, which induces expression of the purine metabolic enzyme;
d) blocking a cellular transcription factor, activated by the DNA tumor virus, which induces expression of the purine metabolic enzyme;
e) inhibiting interaction of a product of the DNA tumor virus with a mammalian cell gene which encodes the purine metabolic enzyme;
f) inhibiting interaction of the DNA tumor virus factor with a mammalian cell gene which encodes the purine metabolic enzyme; and
g) inhibiting interaction of the DNA tumor virus with a cellular protein, the interaction resulting in regulation of activity of the purine metabolic enzyme in the mammalian cell.

9. Isolated DNA consisting essentially of: (a) the 5' region of the human brain creatine kinase gene, or a functional equivalent thereof, or (b) the promoter region of the human brain creatine kinase gene, or a functional equivalent thereof, e.g. having the sequence: GCCCCTTAAGAGCCGCGGAGCGCGGAGCGGCC, or a functional equivalent thereof.

10. A method of regulating induction by a virus of a cellular gene encoding a product which is involved in adenosine metabolism, comprising altering the ability of a product of the virus to act upon the 5' noncoding region of the cellular gene, wherein for example (a) the product of the virus is an oncogenic product and the 5' region of the cellular gene is the promoter region, and/or (b) the virus is an adenovirus and the cellular gene is selected from the group consisting of the human creatine kinase gene and the human adenosine deaminase gene.

11. A method of disrupting the ability of a viral product to induce expression of an enzyme which participates in a pathway involved in regulating cellular nucleotide levels, comprising interfering with the ability of the viral product to act upon the 5' region of a gene encoding the enzyme, wherein for example (a) the viral product is an oncogenic product, the enzyme is selected from the group consisting of creatine kinase and adenosine deaminase, and the nucleotide is adenoside, and/or (b) the viral product is selected from the group consisting of: the oncogenic product of the domain 2 of the E1a region of an adenovirus and amino acids 2-40 of the oncogenic product E1a of adenovirus.

12. A method of inhibiting in a cell induction by an adenovirus of a gene encoding an enzyme which participates in regulating cellular adenosine levels, comprising interfering with interaction of a product of the adenovirus with the 5' region of the gene, wherein the gene is for example selected from the group consisting of the human creatine kinase gene and the human adenosine deaminase gene.

13. A method of reducing expression from the promoter of a mammalian gene encoding an enzyme which participates in cellular energy metabolism, expression from said promoter being induced by a viral product, comprising interfering with the ability of said viral product to act upon said promoter, wherein for example (a) the ability of said viral product to act upon said promoter is interfered with by inhibiting production of said viral product or by blocking said viral product in such a manner that said viral product cannot induce expression from said promoter, and/or (b) the viral product is an oncogenic product of an adenovirus.

14. A method of reducing expression in a cell from the promoter of the human creatine kinase gene, comprising interfering with the ability of a viral product to induce expression from said promoter, wherein for example the viral product is an oncogenic product of an adenovirus, the oncogenic product for example being encoded by transforming domain 2 or the first 40 amino acids of the E1a region of an adenovirus.

15. A method of inhibiting creatine kinase activity in a cell, comprising introducing into the cell a substrate analogue which interferes with creatine kinase activity by competing with creatine phosphate or with creatine for interaction with creatine kinase, or a composition comprising an interfering substance capable of altering the activity of an enzyme which participates in an adenosine metabolic pathway, the interfering substance for example being capable of altering the activity of creatine kinase and wherein for example : (a) the interfering substance is creatine or creatine phosphate, and/or (b) the interfering substance is capable of altering the activity of adenosine deaminase.
